(19) **Europäisches Patentamt / European Patent Office / Office européen des brevets**

(11) **EP 4 026 894 A1**

(12) **EUROPEAN PATENT APPLICATION**
published in accordance with Art. 153(4) EPC

(43) Date of publication:
**13.07.2022  Bulletin 2022/28**

(21) Application number: **20859828.4**

(22) Date of filing: **04.09.2020**

(51) International Patent Classification (IPC):
***C12M 3/02*** (2006.01)

(52) Cooperative Patent Classification (CPC):
**C12M 3/02**

(86) International application number:
**PCT/CN2020/113460**

(87) International publication number:
**WO 2021/043257 (11.03.2021 Gazette 2021/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **06.09.2019   CN 201910841620**

(71) Applicant: **Zhejiang JYSS Bioengineering Co., Ltd.**
**Huzhou, Zhejiang 313000 (CN)**

(72) Inventors:
• **LUO, Shun**
**Huzhou, Zhejiang 313000 (CN)**

• **HU, Fulin**
**Huzhou, Zhejiang 313000 (CN)**
• **YUAN, Shiping**
**Huzhou, Zhejiang 313000 (CN)**
• **HOU, Jue**
**Huzhou, Zhejiang 313000 (CN)**
• **SONG, Jinpei**
**Huzhou, Zhejiang 313000 (CN)**

(74) Representative: **Piotrowicz, Pawel Jan Andrzej et al**
**Venner Shipley LLP**
**Byron House**
**Cambridge Business Park**
**Cowley Road**
**Cambridge CB4 0WZ (GB)**

(54)  **BIOREACTOR SYSTEM AND APPLICATION THEREOF**

(57)    The present application relates to a bioreactor system for culturing cells, especially cells without cell walls; the bioreactor system includes: a container containing a hollow cylinder with a diameter of D1 and a height of H1, and a hollow circular truncated cone with an upper diameter of D2, a lower diameter of D3, and a height of H2, where the hollow cylinder is connected to a top surface of the hollow circular truncated cone, and D1 = D2; an oscillator, configured to cause the container to make an eccentric motion according to a certain eccentricity and rotational speed; a ventilation device, configured to introduce an oxygen-containing gas from an upper portion of the container to the inside of the container, and a culture solution filled in the container, of which a top surface is exposed to the oxygen-containing gas; where the oscillator is configured to maintain the eccentric motion of the container, such that a ratio of the total liquid surface area to the volume (S/V) of the culture solution when in a steady state of motion is 5.65 or more, the turbulence kinetic energy is 2.73E-03 $m^2/s^2$ or more, and the flow field shear rate is 20.27/s or less, where the total liquid surface area is the sum of the contact area between the culture solution and the reactor wall surface and the contact area between the top surface and the gas.

FIG. 1

**Description**

**TECHNICAL FIELD**

The present application relates to a bioreactor system for culturing cells, especially cells without cell walls, e.g., animal cells, and a method for culturing cells using the bioreactor system. The bioreactor system related in the present application can achieve high-cell viability cell culture and proliferation.

**BACKGROUND OF THE INVENTION**

Products expressed by mammalian cells have possessed absolute advantages in bio-pharmaceutical industry, and become a mainstream and trend in bio-pharmaceuticals. The production line and cell culture process with a bioreactor as a main body are hardware driving force to push the innovation of large-scale culture technologies and process improvement, and are also basics to promote the production efficiency, production scale and product quality of biological products. The existing commercialized animal cell bioreactors at home and abroad include stirred bioreactors, hollow fiber bioreactors, airlift bioreactors and bag-type bioreactors.

One the one hand, because animal cells are free of cell walls and thus, are more sensitive to the culture environment, such as shear force and osmotic pressure, the reactor is required to have high oxygen transfer efficiency, good mixing properties and low shear effect. Therefore, the common problem mainly solved by the bioreactor as a key equipment of animal cell culture is to make the reactor possessing low shear effect, good mass transfer and mixing effects according to the requirements of cell growth. Oxygen supply and culture solution mixing in most of the existing bioreactors are achieved by bottom ventilation and mechanical stirring. Therefore, the strong shear force, high-concentration oxygen bubbles and bubble breakage caused thereby will cause heavy damages on animals and result in a great decrease of cell culture density.

On the other hand, studies on equipment development and biological reaction process of a bioreactor need to be explored and tested in small-sized equipment firstly, and then gradually scaled to larger-sized equipment for process scale-up experiments and commercialized production. However, the data, processes and rules obtained in scientific experiments of small-sized reactors in practice cannot be often completely achieved in large-scale reactors similarly or better. Therefore, the size design of a bioreactor cannot satisfy the culture demands in industrialization scale for animal cells via simple proportional amplification. Currently, most of the amplification methods based on some rules established by the operating experience of the existing bioreactors are qualitative; there are only some simple and general quantitative concepts; and the amplification ratio is usually small and not enough accurate. For the purpose, a bioreactor further needs to be designed and scaled up on the basis of the experience and practical principles before not obtaining the integrated analysis of the theory system.

**SUMMARY OF THE INVENTION**

In view of the above problems, the inventor of the present application performs an analogue simulation on a bioreactor under working conditions through the Computational Fluid Dynamics (CFD), thus exploring a theoretical bioreactor model capable of satisfying the demands for different scales of culture, and verifying the same in practical cell culture. The theoretical model is utilized to obtain a bioreactor system satisfying the demands for different scales of animal cell culture and proliferation; and the animal cell viability is allowed to keep 85.0% above. CFD refers that a governing equation of fluid mechanics is solved in a computer through a numerical method, thus predicting the flow of a flow field. Currently, lots of commercial CFD software have been published, such as, FLUENT, CFD-ACE+ (CFDRC), Phoenics, CFX and Star-cd. A person skilled in the art knows well that CFD software is used for flow field simulation.

On the one hand, the present application relates to a bioreactor system for culturing cells, especially cells without cell walls; the bioreactor system includes:

- a container, including a hollow cylinder with a diameter of D1 and a height of H1, and a hollow circular truncated cone with an upper diameter of D2, a lower diameter of D3, and a height of H2, where the hollow cylinder is connected to a top surface of the hollow circular truncated cone, and D1 = D2;
- an oscillator, configured to cause the container to make an eccentric motion according to a certain eccentricity and rotational speed;
- a ventilation device, configured to introduce an oxygen-containing gas from an upper portion of the container to the inside of the container, and
- a culture solution filled in the container, of which a top surface is exposed to the oxygen-containing gas;

where the oscillator is configured to maintain the eccentric motion of the container, such that a ratio of the total liquid surface area to the volume (S/V) of the culture solution in a steady state of motion is 5.65 or more, a turbulence kinetic energy is 2.73E-03 $m^2/s^2$ or more, and a flow field shear rate is 20.27/s or less, where the total liquid surface area is the sum of the contact area between the culture solution and the inner wall surface of the container and the contact area between the culture solution and the gas in the container. In some embodiments, the S/V value, turbulence kinetic energy and flow field shear rate is obtained by CFD simulation.

The CFD simulation may be achieved by FLUENT software. The S/V value of the culture solution in a steady state of motion is related to the shape of the container, the volume V of the culture solution, the rotational speed and eccentricity R of the container, and may be obtained by CFD simulation. The shear rate produced in the container is related to the shape, rotational speed and eccentricity R of the container, and may be obtained by CFD simulation. The turbulence kinetic energy in the container is related to the rotational speed and eccentricity R of the container, and may be obtained by CFD simulation.

The bioreactor system may further contain a disposable culture bag which is disposed in the container and used for holding the culture solution; the disposable culture bag has a multifunctional cover plate, and the multifunctional cover plate is connected to the top of the culture bag to seal the culture bag, and provided with a plurality of connecting holes leading to the inside of the disposable culture bag. The disposable culture bag may be a flexible culture bag or made of a hard material, and has a shape corresponding to the container when expanded. The disposable culture bag may be provided with a device which may fix the same into the container. The connecting holes on the above multifunctional cover plate have good airtightness, and may be switched into exploring electrodes, conduits and the like when necessary. In some embodiments, each connecting hole is sealed through screw threads with good air-tightness. In some embodiments, the exploring electrodes are switched via any connecting hole to real-timely monitor the environmental parameters, such as temperature, dissolved oxygen and pH of the cell culture process. In some embodiments, conduits are switched via a connecting hole to perform various operations, such as cell culture inoculation, addition of culture solution, sampling, recovery, good yield, and air exchange, thereby further optimizing the culture conditions and improving the cell culture density. Meanwhile, each connecting hole capable of being applied in multifunctional cover plates of various disposable culture bags uses unified screw interfaces and has good air tightness such that optional operations may be flexibly performed according to the cell culture requirements. Unnecessary connecting holes in a specific culture process may be readily sealed.

In some embodiments, in the bioreactor system, the container has a D1 and a D2 of 400-4000 mm, a D3 of 40-400 mm, a H1 of 100-1500 mm, and a H2 of 40-1200 mm.

In some embodiments, the value of D1:D3 or D2:D3 is any value within an interval of about 5 to about 16, or any value within an interval of 5 to 16. In some embodiments, the value of D1:D3 or D2:D3 is about 5, about 7.37, about 7.89, about 8.89, about 10.27 or about 15.77. In some embodiments, the value of D1:D3 or D2:D3 is about 5, 7.37, 7.89, 8.89, 10.27 or 15.77. The term "about" herein means a scope of the value ±20%, ±18%, ±15%, ±12%, ±10%, ±9%, ±8%, ±7%, ±6%, ±5%, ±4%, ±3%, ±2%, ±1% or ±0.5%; the scope includes end-point values of the scope and any value within the scope.

In some embodiments, the value of D1:H1 or D2:H1 is any value within an interval of about 2 to about 5, or any value within an interval of 2 to 5. In some embodiments, the value of D1:H1 or D2:H1 is about 2.68, about 3, about 3.55, about 3.74, about 3.66 or about 3.46. In some embodiments, the value of D1:H1 or D2:H1 is 2.68, 3, 3.55, 3.74, 3.66 or 3.46.

In some embodiments, the value of D1:H2 or D2:H2 is any value within an interval of about 4 to about 5, or any value within an interval of 4 to 5. In some embodiments, the value of D1:H2 or D2:H2 is about 4.08, about 4.94, about 4.95, about 4.87, about 4.20 or about 4.51. In some embodiments, the value of D1:H2 or D2:H2 is 4.08, 4.94, 4.95, 4.87, 4.20 or 4.51.

In some embodiments, the container in the bioreactor system has a D1 and a D2 of about 400 mm to about 2997 mm, a D3 of about 80 mm to about 190 mm, a H1 of about 149 mm to about 867 mm, a H2 of about 98 mm to about 664 mm, and contains about 5 L to about 3000 L cell culture solution; and the oscillator has a rotational speed of about 55 rpm to about 24 rpm, and an eccentricity of about 30 mm to about 65 mm. In some embodiments, the container in the bioreactor system has a D1 and a D2 of 400-2997 mm, a D3 of 80-190 mm, a H1 of 149-867 mm, a H2 of 98-664 mm, and contains 5-3000 L cell culture solution; and the oscillator has a rotational speed of 55-24 rpm, and an eccentricity of 30-65 mm.

In some embodiments, the container in the bioreactor system has a D1 and a D2 of about 400 mm, a D3 of about 80 mm, a H1 of about 149 mm, a H2 of about 98 mm, and contains about 5 L cell culture solution; and the oscillator has a rotational speed of about 55 rpm, and an eccentricity of about 30 mm. In some embodiments, the container in the bioreactor system has a D1 and a D2 of 400 mm, a D3 of 80 mm, a H1 of 149 mm, a H2 of 98 mm, and contains about 5 L cell culture solution; and the oscillator has a rotational speed of 55 rpm, and an eccentricity of 30 mm.

In some embodiments, the container in the bioreactor system has a D1 and a D2 of about 840 mm, a D3 of about 114 mm, a H1 of about 280 mm, a H2 of about 170 mm, and contains about 50 L cell culture solution; and the oscillator has a rotational speed of about 40 rpm, and an eccentricity of about 40 mm. In some embodiments, the container in the bioreactor system has a D1 and a D2 of 840 mm, a D3 of 114 mm, a H1 of 280 mm, a H2 of 170 mm, and contains 50 L cell culture solution; and the oscillator has a rotational speed of 40 rpm, and an eccentricity of 40 mm.

In some embodiments, the container in the bioreactor system has a D1 and a D2 of about 840 mm, a D3 of about 114 mm, a H1 of about 280 mm, a H2 of about 170 mm, and contains about 18 L cell culture solution; and the oscillator has a rotational speed of about 37 rpm to about 39 rpm, and an eccentricity of about 40 mm. In some embodiments, the container in the bioreactor system has a D1 and a D2 of 840 mm, a D3 of 114 mm, a H1 of 280 mm, a H2 of 170 mm, and contains about 18 L cell culture solution; and the oscillator has a rotational speed of 37-39 rpm, and an eccentricity of 40 mm.

In some embodiments, the container in the bioreactor system has a D1 and a D2 of about 840 mm, a D3 of about 114 mm, a H1 of about 280 mm, a H2 of about 170 mm, and contains about 18 L cell culture solution; and the oscillator has a rotational speed of about 39 rpm, and an eccentricity of about 40 mm. In some embodiments, the container in the bioreactor system has a D1 and a D2 of 840 mm, a D3 of 114 mm, a H1 of 280 mm, a H2 of 170 mm, and contains 18 L cell culture solution; and the oscillator has a rotational speed of 39 rpm, and an eccentricity of 40 mm.

In some embodiments, the container in the bioreactor system has a D1 and a D2 of about 1500 mm, a D3 of about 190 mm, a H1 of about 422 mm, a H2 of about 303 mm, and contains about 18 L cell culture solution; and the oscillator has a rotational speed of about 37 rpm to about 39 rpm, and an eccentricity of about 40 mm. In some embodiments, the container in the bioreactor system has a D1 and a D2 of 1500 mm, a D3 of 190 mm, a H1 of 422 mm, a H2 of 303 mm, and contains about 18 L cell culture solution; and the oscillator has a rotational speed of 37-39 rpm, and an eccentricity of 40 mm.

In some embodiments, the container in the bioreactor system has a D1 and a D2 of about 1500 mm, a D3 of about 190 mm, a H1 of about 422 mm, a H2 of about 203 mm, and contains about 200 L cell culture solution; and the oscillator has a rotational speed of about 30 rpm, and an eccentricity of about 60 mm. In some embodiments, the container in the bioreactor system has a D1 and a D2 of 1500 mm, a D3 of 190 mm, a H1 of 422 mm, a H2 of 203 mm, and contains 200 L cell culture solution; and the oscillator has a rotational speed of 30 rpm, and an eccentricity of 60 mm.

In some embodiments, the container in the bioreactor system has a D1 and a D2 of about 1500 mm, a D3 of about 190 mm, a H1 of about 422 mm, a H2 of about 203 mm, and contains about 205 L cell culture solution; and the oscillator has a rotational speed of about 30 rpm, and an eccentricity of about 60 mm. In some embodiments, the container in the bioreactor system has a D1 and a D2 of 1500 mm, a D3 of 190 mm, a H1 of 422 mm, a H2 of 203 mm, and contains 205 L cell culture solution; and the oscillator has a rotational speed of 30 rpm, and an eccentricity of 60 mm.

In some embodiments, the container in the bioreactor system has a D1 and a D2 of about 1500 mm, a D3 of about 190 mm, a H1 of about 422 mm, a H2 of about 203 mm, and contains about 250 L cell culture solution; and the oscillator has a rotational speed of about 30 rpm, and an eccentricity of about 60 mm. In some embodiments, the container in the bioreactor system has a D1 and a D2 of 1500 mm, a D3 of 190 mm, a H1 of 422 mm, a H2 of 203 mm, and contains 250 L cell culture solution; and the oscillator has a rotational speed of 30 rpm, and an eccentricity of 60 mm.

In some embodiments, the container in the bioreactor system has a D1 and a D2 of about 1690 mm, a D3 of about 190 mm, a H1 of about 452 mm, a H2 of about 347 mm, and contains about 500 L cell culture solution; and the oscillator has a rotational speed of about 28 rpm, and an eccentricity of about 65 mm. In some embodiments, the container in the bioreactor system has a D1 and a D2 of 1690 mm, a D3 of 190 mm, a H1 of 452 mm, a H2 of 347 mm, and contains 500 L cell culture solution; and the oscillator has a rotational speed of 28 rpm, and an eccentricity of 65 mm.

In some embodiments, the container in the bioreactor system has a D1 and a D2 of about 1952 mm, a D3 of about 190 mm, a H1 of about 533 mm, a H2 of about 465 mm, and contains about 1200 L cell culture solution; and the oscillator has a rotational speed of about 25 rpm, and an eccentricity of about 65 mm. In some embodiments, the container in the bioreactor system has a D1 and a D2 of 1952 mm, a D3 of 190 mm, a H1 of 533 mm, a H2 of 465 mm, and contains 1200 L cell culture solution; and the oscillator has a rotational speed of 25 rpm, and an eccentricity of 65 mm.

In some embodiments, the container in the bioreactor system has a D1 and a D2 of about 1952 mm, a D3 of about 190 mm, a H1 of about 533 mm, a H2 of about 465 mm, and contains about 250 L cell culture solution; and the oscillator has a rotational speed of about 30 rpm, and an eccentricity of about 65 mm. In some embodiments, the container in the bioreactor system has a D1 and a D2 of 1952 mm, a D3 of 190 mm, a H1 of 533 mm, a H2 of 465 mm, and contains 250 L cell culture solution; and the oscillator has a rotational speed of 30 rpm, and an eccentricity of 65 mm.

In some embodiments, the container in the bioreactor system has a D1 and a D2 of about 1952 mm, a D3 of about 190 mm, a H1 of about 533 mm, a H2 of about 465 mm, and contains about 330 L cell culture solution; and the oscillator has a rotational speed of about 30 rpm, and an eccentricity of about 65 mm. In some embodiments, the container in the bioreactor system has a D1 and a D2 of 1952 mm, a D3 of 190 mm, a H1 of 533 mm, a H2 of 465 mm, and contains 330 L cell culture solution; and the oscillator has a rotational speed of 30 rpm, and an eccentricity of 65 mm.

In some embodiments, the bioreactor system having a D1 and a D2 of about 2997 mm, a D3 of about 190 mm, a H1 of about 867 mm, a H2 of about 664 mm, and containing about 3000 L cell culture solution is used for cell culture; and a rotational speed of the oscillator is configured about 24 rpm, and an eccentricity is configured about 65 mm. In some embodiments, the bioreactor system having a D1 and a D2 of 2997 mm, a D3 of 190 mm, a H1 of 867 mm, a H2 of 664 mm, and containing 3000 L cell culture solution is used for cell culture; and a rotational speed of the oscillator is configured about 24 rpm, and an eccentricity is configured about 65 mm.

In some embodiments, the container in the bioreactor system has a D1 and a D2 of 400 mm, a D3 of 80 mm, a H1 of 149 mm, a H2 of 98 mm, and contains 5 L cell culture solution; and the oscillator has a rotational speed of 55 rpm, and an eccentricity of 30 mm. The culture solution obtained by CFD simulation in a steady state of motion has a S/V value of X, a turbulence kinetic energy of X $m^2/s^2$ and a flow field shear rate of X/s. In some embodiments, the culture solution obtained by CFD simulation of the bioreactor system in a steady state of motion in has a S/V value of 50.48, a turbulence kinetic energy of 2.73E-03 $m^2/s^2$ and a flow field shear rate of 10.18/s.

In some embodiments, the container in the bioreactor system has a D1 and a D2 of 840 mm, a D3 of 114 mm, a H1 of 280 mm, a H2 of 170 mm, and contains 50 L cell culture solution; and the oscillator has a rotational speed of 40 rpm, and an eccentricity of 40 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 26.61, a turbulence kinetic energy of 5.29E-03 $m^2/s^2$ and a flow field shear rate of 7.02/s.

In some embodiments, the container in the bioreactor system has a D1 and a D2 of 840 mm, a D3 of 114 mm, a H1 of 280 mm, a H2 of 170 mm, and contains 18 L cell culture solution; and the oscillator has a rotational speed of 37-39 rpm, and an eccentricity of 40 mm. The culture solution obtained by CFD simulation in a steady state of motion has a S/V value of X, a turbulence kinetic energy of X $m^2/s^2$ and a flow field shear rate of X/s. In some embodiments, the oscillator in the bioreactor system has a rotational speed of 37 rpm, a S/V value obtained by CFD simulation in a steady state of motion of 43.34, a turbulence kinetic energy of 4.71E-03 $m^2/s^2$ and a flow field shear rate of 6.8056/s. In some embodiments, the container in the bioreactor system has a D1 and a D2 of 840 mm, a D3 of 114 mm, a H1 of 280 mm, a H2 of 170 mm, and contains 18 L cell culture solution; and the oscillator has a rotational speed of 39 rpm, and an eccentricity of 40 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 47.89, a turbulence kinetic energy of 5.35E-03 $m^2/s^2$ and a flow field shear rate of 7.1149/s.

In some embodiments, the container in the bioreactor system has a D1 and a D2 of 1500 mm, a D3 of 190 mm, a H1 of 422 mm, a H2 of 303 mm, and contains 200 L cell culture solution; and the oscillator has a rotational speed of 30 rpm, and an eccentricity of 60 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 18.46, a turbulence kinetic energy of 9.91E-03 $m^2/s^2$ and a flow field shear rate of 5.9707/s. In some embodiments, the container in the bioreactor system has a D1 and a D2 of 1500 mm, a D3 of 190 mm, a H1 of 422 mm, a H2 of 303 mm, and contains 205 L cell culture solution; and the oscillator has a rotational speed of 30 rpm, and an eccentricity of 60 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 18.24, a turbulence kinetic energy of 9.89E-03 $m^2/s^2$ and a flow field shear rate of 5.7476/s. In some embodiments, the container in the bioreactor system has a D1 and a D2 of 1500 mm, a D3 of 190 mm, a H1 of 422 mm, a H2 of 303 mm, and contains 250 L cell culture solution; and the oscillator has a rotational speed of 30 rpm, and an eccentricity of 60 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 15.98, a turbulence kinetic energy of 9.30E-03 $m^2/s^2$ and a flow field shear rate of 5.4623/s.

In some embodiments, the container in the bioreactor system has a D1 and a D2 of 1690 mm, a D3 of 190 mm, a H1 of 452 mm, a H2 of 347 mm, and contains 500 L cell culture solution; and the oscillator has a rotational speed of 28 rpm, and an eccentricity of 65 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 10.61, a turbulence kinetic energy of 7.59E-03 $m^2/s^2$ and a flow field shear rate of 4.66/s.

In some embodiments, the container in the bioreactor system has a D1 and a D2 of 1952 mm, a D3 of 190 mm, a H1 of 533 mm, a H2 of 465 mm, and contains 1200 L cell culture solution; and the oscillator has a rotational speed of 25 rpm, and an eccentricity of 65 mm. The culture solution obtained by CFD simulation in steady state has a S/V value of 6.60, a turbulence kinetic energy of 9.30E-03 $m^2/s^2$ and a flow field shear rate of 4.42/s. In some embodiments, the container in the bioreactor system has a D1 and a D2 of 1952 mm, a D3 of 190 mm, a H1 of 533 mm, a H2 of 465 mm, and contains 250 L cell culture solution; and the oscillator has a rotational speed of 30 rpm, and an eccentricity of 65 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 20.96, a turbulence kinetic energy of 1.46E-02 $m^2/s^2$

and a flow field shear rate of 6.0922/s. In some embodiments, the container in the bioreactor system has a D1 and a D2 of 1952 mm, a D3 of 190 mm, a H1 of 533 mm, a H2 of 465 mm, and contains 330 L cell culture solution; and the oscillator has a rotational speed of 30 rpm, and an eccentricity of 65 mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 18.09, a turbulence kinetic energy of 1.52E-02 $m^2/s^2$ and a flow field shear rate of 6.3173/s.

In some embodiments, the container in the bioreactor system has a D1 and a D2 of 2997 mm, a D3 of 190 mm, a H1 of 867 mm, a H2 of 664 mm, and contains 3000 L cell culture solution; and the oscillator has a rotational speed of 24 rpm, and an eccentricity of 65mm. The culture solution obtained by CFD simulation in steady state has a S/V value of X, a turbulence kinetic energy of X $m^2/s^2$ and a flow field shear rate of X/s. In some embodiments, the culture solution obtained by CFD simulation of the bioreactor system in a steady state of motion has a S/V value of 5.65, a turbulence kinetic energy of 2.48E-02 $m^2/s^2$ and a flow field shear rate of 3.98/s.

On the other hand, the present application relates to a method for culturing cells, especially cells without cell walls using the bioreactor system; the bioreactor system contains:

- a container, including a hollow cylinder with a diameter of D1 and a height of H1, and a hollow circular truncated cone with an upper diameter of D2, a lower diameter of D3, and a height of H2, where the hollow cylinder is connected to a top surface of the hollow circular truncated cone, and D1 = D2;
- an oscillator, configured to cause the container to make an eccentric motion according to a certain eccentricity and rotational speed;
- a ventilation device, configured to introduce an oxygen-containing gas from an upper portion of the container to the inside of the container, and
- a culture solution filled in the container, of which a top surface is exposed to the oxygen-containing gas;

the cell culture method includes:

- calculating a rotational speed and an eccentricity of the oscillator required on the condition that a ratio of the total liquid surface area to the volume (S/V) of the culture solution is 5.65 or more, a turbulence kinetic energy is 2.73E-03 $m^2/s^2$ or more, and a flow field shear rate is 20.27/s or less when the culture solution achieves a steady state of eccentric motion through CFD simulation according to the shape of the bioreactor system and the volume of the culture solution, wherein the total liquid surface area is the sum of the contact area between the culture solution and the reactor wall surface and the contact area between the top surface and the gas; and
- adding the culture solution in the bioreactor system and inoculating cells, and configuring the oscillator according to the calculated rotational speed and eccentricity of the oscillator, and performing cell culture.

The CFD simulation may be performed by FLUENT software.

According to the total working power of the bioreactor system, the rotational speed of the oscillator decreases with the increase of the container volume. According to the working efficiency of the bioreactor system, the eccentricity of the oscillator increases with the increase of the container volume.

For example, in a bioreactor system having a maximum working volume of 5 L, the rotational speed of the oscillator may be configured within a scope of 45-70 rpm and the eccentricity thereof may be configured about 30 mm; in a bioreactor system having a maximum working volume of 50 L, the rotational speed of the oscillator may be configured within a scope of 40-60 rpm and the eccentricity thereof may be configured about 40 mm; in a bioreactor system having a maximum working volume of 500 L, the rotational speed of the oscillator may be configured within a scope of 25-30 rpm and the eccentricity thereof may be configured about 65 mm; in a bioreactor system having a maximum working volume of 1200 L, the rotational speed of the oscillator may be configured within a scope of 20-30 rpm and the eccentricity thereof may be configured about 65 mm; and in a bioreactor system having a maximum working volume of 3000 L, the rotational speed of the oscillator may be configured within a scope of 24-26 rpm and the eccentricity thereof may be configured about 65 mm.

The bioreactor system may further contain a disposable culture bag which is disposed in the container and used for holding the culture solution; the disposable culture bag has a multifunctional cover plate, and the multifunctional cover plate is connected to the top of the culture bag to seal the culture bag, and provided with a plurality of connecting holes leading to the inside of the disposable culture bag. The disposable culture bag may be a flexible culture bag or made of a hard material, and has a shape corresponding to the container when expanded. The disposable culture bag may be provided with a device which may fix the same into the container. The connecting holes on the above multifunctional cover plate have good airtightness, and may be switched into exploring electrodes, conduits and the like when necessary. In some embodiments, each connecting hole is sealed through screw threads with good air-tightness. In some embodiments, the exploring electrodes are switched via any connecting hole to real-timely monitor the environmental parameters, such as temperature, dissolved oxygen and pH of the cell culture process. In some embodiments, conduits are switched via a connecting hole to perform various operations, such as cell culture inoculation, addition of culture solution, sampling, recovery, good yield, and air exchange, thereby further optimizing the culture conditions and improving the cell culture density. Meanwhile, each connecting hole capable of being applied in multifunctional cover plates of various disposable culture bags uses unified screw interfaces and has good air tightness such that optional operations may be flexibly performed according to the cell culture requirements. Unnecessary connecting holes in a specific culture process may be readily sealed.

In one embodiment, the bioreactor system having a D1 and a D2 of 400 mm, a D3 of 80 mm, a H1 of 149 mm, a H2 of 98 mm, and containing 5 L cell culture solution is used for cell culture; and a rotational speed of the oscillator is configured about 55 rpm, and an eccentricity is configured about 30 mm.

In one embodiment, the bioreactor system having a D1 and a D2 of 840 mm, a D3 of 114 mm, a H1 of 280 mm, a H2 of 170 mm, and containing 50 L cell culture solution is used for cell culture; and a rotational speed of the oscillator is configured about 40 rpm, and an eccentricity is configured about 40 mm.

In one embodiment, the container in the bioreactor system has a D1 and a D2 of 1500 mm, a D3 of 190 mm, a H1 of 422 mm, a H2 of 303 mm, and contains about 18 L cell culture solution; and the oscillator has a rotational speed of 37-39 rpm, and an eccentricity of 40 mm.

In one embodiment, the container in the bioreactor system has a D1 and a D2 of 1500 mm, a D3 of 190 mm, a H1 of 422 mm, a H2 of 203 mm, and contains 200 L cell culture solution; and the oscillator has a rotational speed of 30 rpm, and an eccentricity of 60 mm. In one embodiment, the container in the bioreactor system has a D1 and a D2 of 1500 mm, a D3 of 190 mm, a H1 of 422 mm, a H2 of 203 mm, and contains 205 L cell culture solution; and the oscillator has a rotational speed of 30 rpm, and an eccentricity of 60 mm. In one embodiment, the container in the bioreactor system has a D1 and a D2 of 1500 mm, a D3 of 190 mm, a H1 of 422 mm, a H2 of 203 mm, and contains 250 L cell culture solution; and the oscillator has a rotational speed of 30 rpm, and an eccentricity of 60 mm.

In one embodiment, the bioreactor system having a D1 and a D2 of 1690 mm, a D3 of 190 mm, a H1 of 452 mm, a H2 of 347 mm, and containing 500 L cell culture solution is used for cell culture; and a rotational speed of the oscillator is configured about 28 rpm, and an eccentricity is configured about 65 mm.

In one embodiment, the bioreactor system having a D1 and a D2 of 1952 mm, a D3 of 190 mm, a H1 of 533 mm, a H2 of 465 mm, and containing 1200 L cell culture solution is used for cell culture; and a rotational speed of the oscillator is configured about 25 rpm, and an eccentricity is configured about 65 mm.

In one embodiment, the container in the bioreactor system has a D1 and a D2 of 1952 mm, a D3 of 190 mm, a H1 of 533 mm, a H2 of 465 mm, and contains 250 L cell culture solution; and the oscillator has a rotational speed of 30 rpm, and an eccentricity of 65 mm.

In one embodiment, the container in the bioreactor system has a D1 and a D2 of 1952 mm, a D3 of 190 mm, a H1 of 533 mm, a H2 of 465 mm, and contains 330 L cell culture solution; and the oscillator has a rotational speed of 30 rpm, and an eccentricity of 65 mm.

In one embodiment, the bioreactor system having a D1 and a D2 of 2997 mm, a D3 of 190 mm, a H1 of 867 mm, a H2 of 664 mm, and containing 3000 L cell culture solution is used for cell culture; and a rotational speed of the oscillator is configured about 24 rpm, and an eccentricity is configured about 65 mm.

In the bioreactor system of this present application, the culture solution produces a lower shear force during the eccentric motion and thus causes less damage on cells, particularly suitable for cells without cell walls, for example, culture of animal cells. Moreover, when the S/V value in steady state and turbulence kinetic energy of the culture solution can be controlled within a certain scope during the eccentric motion; the efficiency of dissolved oxygen and diffused oxygen is higher, which is thus free of oxygen toxicity when the oxygen concentration is too high or limited cell growth and proliferation when the oxygen concentration is too low due to uneven oxygen distribution, and can support the high-density growth of cells better. When the method of the present application is used for cell culture, the cell growth and proliferation efficiency are higher regardless of a small-sized culture or large-sized culture; moreover, the cell viability is kept 90.0%, 95.0% and even 99.0% above. Especially, when a disposable culture bag is combined in use, the present application can avoid cross contamination, shorten the processing period between batches, and need no complex pipe and other ancillary facilities, and free of cleaning, sterilization and verification, thereby greatly improving the working efficiency.

## BRIEF DESCRIPTION OF THE DRAWINGS

FIG. 1 shows a structure diagram of a container of a bioreactor system in the present application.
FIG. 2 is a schematic diagram showing a movement locus of any point on the container during eccentric motion.
FIG. 3 is a simulated diagram showing a change of free liquid level when CUR300L rotates a round at a working volume of 300 L and a rotational speed of 28 rpm.

FIG. 4 is a simulated diagram showing a change of free liquid level when CUR300L rotates a round at a working volume of 300 L and a rotational speed of 30 rpm.

FIG. 5 is a simulated diagram showing a change of free liquid level when CUR300L rotates a round at a working volume of 175 L and a rotational speed of 28 rpm.

FIG. 6 is a simulated diagram showing a change of free liquid level when CUR300L rotates a round at a working volume of 175 L and a rotational speed of 30 rpm.

**Detailed Embodiments of the Invention**

When cells, in particular to cells without cell walls, for example, animal cells are cultured, the most ideal condition is that the reactor causes a little physical damage on cells, and the reactor can timely provide enough oxygen to support the cell growth and proliferation. The bioreactor system is required to produce less shear force and provide a higher efficiency of dissolved oxygen and diffused oxygen. The objective of the present application is to provide a bioreactor system having the effect mentioned above.

Studies on equipment development and biological reaction process of a bioreactor need to be explored and tested in small-sized equipment firstly, and then gradually scaled to larger-sized equipment for process scale-up experiments and commercialized production. However, the data, processes and rules obtained in a small-sized reactor by scientific experiments in practice cannot be completely achieved in a large-scale reactor similarly or better. Therefore, the size design of a bioreactor cannot satisfy the culture demands in industrialization scale for animal cells via simple proportional amplification.

Therefore, the inventor of the present application performs analogue simulation on various sizes of bioreactors in working conditions through the Computational Fluid Dynamics (CFD), and is focused on surveying the relative position change, gas-liquid interface area, total liquid surface area, liquid turbulence kinetic energy, dissipation rate of turbulence kinetic energy, turbulence intensity, shear force change and the like of the culture solution in 5 types of reactors with different volume (CUR2.5L, CUR50L, CUR300L, CUR1200L and CUR3000L) in the working condition that the oscillator makes an eccentric motion along with the vertical axis. When some parameters are selected and combined and applied to practical cell culture, it is found that both 5 L and 1200 L bioreactor systems can achieve the steady growth of cells; and the cell viability is 90.0%, 95.0% and even 99% above.

The bioreactor system of the present application mainly includes a container and an oscillator. The container consists of an upper hollow cylinder and a lower hollow circular truncated cone, and the container makes a uniform circular motion around an equal radius of eccentricity. When the ratio S/V of the total liquid surface area of the culture solution to the volume when the eccentric motion is in a steady state, the turbulence kinetic energy and the flow field shear rate are controlled within a certain scope, the steady growth of cells may be achieved and the cell viability is kept at a high level.

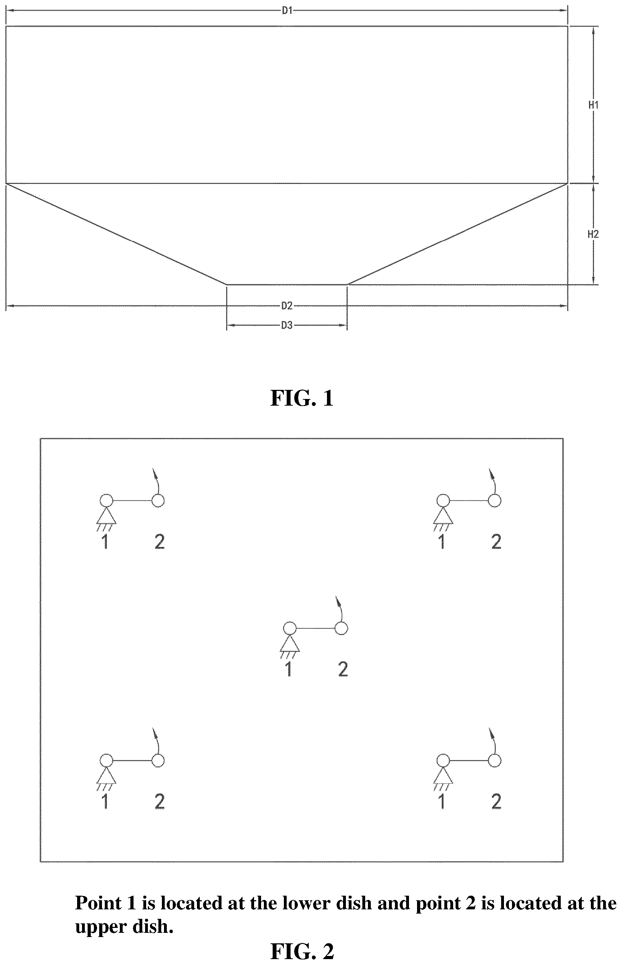

The oscillator makes an "eccentric motion", which refers that a motor in an oscillator drives a belt pulley to rotate simultaneously via a belt, the belt pulley is connected with three cranks to drive a mobile platform to move; each point on the mobile platform makes a uniform circular motion around an equal radius of eccentricity, and has the same movement locus; and each point has the same speed and accelerated speed at any moment. The container of the bioreactor system is fixed on the mobile platform of the oscillator. The container makes a translational motion on a horizontal plane; the movement loci of other parts can be determined as long as the movement locus of any point on the container is determined, as shown in FIG. 2.

"Dissolved oxygen" refers that the top surface of the culture solution contacts an oxygen-containing gas to bring the gas into the culture solution; and the oscillator drives the container of the bioreactor system to make an eccentric motion such that the culture solution causes a slow movement to continuously flush the surface of the container, thus manufacturing soluble nanoscale microbubbles and carrying the gas into the culture solution. "Diffused oxygen" refers that the oxygen-containing gas brought into liquid via turbulent diffusion and the like of the culture solution is transferred to the inside of the culture solution.

"Total liquid surface area" of the culture solution refers to the sum of the contact area between a culture solution and the wall surface of a reactor and the contact area between the culture solution and gas, and is abbreviated S; a ratio of S to the volume of the culture solution is called a S/V ratio. In this text, the contact area between the culture solution and the gas is abbreviated A. The top surface of the culture solution directly contacts with oxygen to dissolve a portion of oxygen. Meanwhile, the update rate of the gas of a liquid film adhered on an inner wall of the reactor container may be different from the main body of a liquid phase. Therefore, the liquid which rotates to the position contacts the liquid film to influence the gas composition of the main body possibly. When the bioreactor system is in an eccentric motion, the culture solution will deviate from the central axis of the reactor container to different extents. When the rotational speed is higher, the centrifugal force on the liquid is larger, and the deviation extent is more obvious, the liquid is extruded more thinly, and the interface in contact with the air concaves more obviously. Accordingly, during the eccentric motion, the contact area between the culture solution and the wall surface of the bioreactor as well as the contact area between the top surface of the culture solution and the gas will be increased to different extents, such that the total liquid surface area and the S/V ratio of the culture solution in a steady state of motion are increased.

In the culture of cells without cell walls, e.g., animal cells, due to the loss of cell walls, the cells are more sensitive to the dynamic environment within a flow field. The mechanical damage caused by an external force on cells is a non-negligible factor in the design and use processes of the animal cell bioreactor. The shear force on cells in the bioreactor is divided into major three categories: a flow field shear force generated by the relative motion inside a culture solution; and the flow field shear force is in direct proportion to a velocity gradient and a kinematic viscosity of the culture solution; a shear force generated when bubbles rise in a culture solution and the surface is broken; and a shear force generated by the collision between cells and the inner wall of a reactor as well as the mutual collision between cells.

When the bioreactor system of the present application is selected, and because a surface oxygen transfer mode is used and there is no bubble sparger, there is precious little bubble generated. Accordingly, the shear force damage caused by bubbles may be ignored. Moreover, the bioreactor system of the present application is a structure of a hollow cylinder plus a hollow circular truncated cone. When the bioreactor system makes an eccentric motion, the walls of the reactor in contact with the culture solution and cells in the bioreactor are very smooth. Therefore, the generated collision shear force is relatively small.

Thereby, the shear force inside the bioreactor of the present application mainly refers to a flow field shear force. The damage of the flow field shear force on cells is closely related to the minimum eddy size generated by fluid turbulence. The culture solution is adhered on a wall surface of a tank to rotate, thus producing a turbulent eddy; and the large eddy gradually develops into a small eddy, and then the small eddy develops into a smaller eddy; and the process is accompanied with energy transfer, namely, the mechanical energy of the culture solution changes to heat energy, thus resulting in the increase of temperature. During the gradient change of the eddy, when the size of the eddy is close to the size of cells, energy is transferred to the cells by the eddy, thus resulting in cell rupture. In the CFD simulation, it is found that the mean shear force or mean shear rate increases with the increase of the rotational speed, but is less influenced by the volume of the culture solution in a same bioreactor; a bioreactor with a larger volume may generate a smaller shear force. When the bioreactor system of the present application is selected, the generated mean shear rate is very low no matter what the shape and specification of the container, the volume of the culture solution, the rotational speed and eccentricity of the oscillator are; and the mean shear rate differs from a shear rate threshold ($391.41s^{-1}$) in water of the Chinese hamster ovary cells (CHO) in an order of magnitude. Therefore, such a parameter, shear force, may be not especially considered when the bioreactor system is configured.

The efficiency of dissolved oxygen and diffused oxygen in the culture solution is related to the surface area, turbulence kinetic energy, turbulence intensity and the like of the culture solution. When the efficiency of dissolved oxygen and diffused oxygen is higher enough to support cell growth and proliferation, it is unnecessary to use a gas with a particularly high oxygen concentration. Based on the previous experience, the gas with a particularly high oxygen concentration, in particular to pure oxygen, is easy to cause oxygen toxicity.

The efficiency of dissolved oxygen in the culture solution is mainly related to a surface area of the oxygen-containing gas in contact with a medium. When the container of the bioreactor system is in an eccentric motion, the top surface of the culture solution as well as the contact surface between the top surface of the culture solution and the container wall may contact the oxygen-containing gas. Therefore, compared with the total surface area or S/V ratio in static state, the total surface area or S/V ratio in a steady state of motion has more reference values. In the CFD simulation, it is found that the S/V ratio in a steady state of motion will be influenced by different specifications of containers, volume of the culture solution, rotational speed of the oscillator and the like. In general, the larger the S/V ratio is, the higher the efficiency of dissolved oxygen is. In case of a lower S/V ratio, the container constructed by the present application may still achieve the effects of steady cell growth and proliferation as well as higher cell viability.

The efficiency of diffused oxygen in the culture solution is mainly related to the turbulence kinetic energy, turbulence intensity and the like of the culture solution.

The motion of fluid micelles may be represented as the superposition of the mean velocity thereof flowing with the main body and the fluctuation velocity of random fluctuation thereof. The random fluctuation of micelles causes turbulent flow. The input power increases with the increase of the rotational speed such that the proportion of the fluid micelles carrying higher energy increases and the number of microscopic eddies capable of generating effective collision for energy transmission increases, namely, manifested as the increase of the mean turbulence kinetic energy. It can be seen from the CFD simulation that in case of a constant working volume, the higher the rotational speed is, the higher the turbulence kinetic energy is. Therefore, the container has better material transfer and mixing effects. Moreover, the random fluctuation of micelles causes turbulent diffusion. There exists violent velocity fluctuation among the micelles; the stronger the interaction is, the more significant the energy transfer and dispersion effect are. Therefore, the stronger the turbulence intensity is, the better the material transfer and mixing effects within a liquid phase are. With the increase of the rotational speed, the turbulence intensity shows an increased trend; the improved rotational speed increases the energy input to fluid, thus resulting in increased kinetic energy of fluid micelles, accelerated fluctuation velocity and correspondingly increased turbulence intensity. The turbulence kinetic energy and turbulence intensity are mainly related to the mean velocity and turbulent fluctuation velocity of fluid, and is in positive correlation to the rotational speed of the oscillator to some extent. One of them may be considered when the efficiency of diffused oxygen in the culture solution is taken into consideration.

By the CFD simulation, a proper volume of the culture solution, rotational speed and eccentricity of the oscillator may be selected directed to a certain bioreactor, thus achieving the effects of small shear effect, and high efficiency of dissolved oxygen and diffused oxygen.

Because the dead weight of the bioreactor system increases with the increase of the volume, a lower rotational speed may be selected to a larger reactor in the premise of considering the total working power of the bioreactor system. In the other aspect, eccentricity needs to be selected according to the volume of the bioreactor from the aspect of working efficiency of the bioreactor. Generally, the eccentricity of the oscillator increases with the increase of the container volume.

For example, in a bioreactor system having a maximum working volume of 5 L, the rotational speed of the oscillator may be configured within a scope of 45-70 rpm and the eccentricity thereof may be configured about 30 mm; in a bioreactor system having a maximum working volume of 50 L, the rotational speed of the oscillator may be configured within a scope of 45-60rpm and the eccentricity thereof may be configured about 40 mm; in a bioreactor system having a maximum working volume of 500 L, the rotational speed of the oscillator may be configured within a scope of 25-30 rpm and the eccentricity thereof may be configured about 65 mm; and in a bioreactor system having a maximum working volume of 1200 L, the rotational speed of the oscillator may be configured within a scope of 20-30 rpm and the eccentricity thereof may be configured about 65 mm.

In the following description, the present application will be further described by detailed examples, but the present application is not limited to the following examples.

### Example 1 Computer analog simulation experiments of the bioreactor

CFD software, FLUENT software was used to simulate the properties of a flow field in a container of a reactor with different working volume at different rotational speed. The present application was focused on surveying the changes of the relative position, gas-liquid interface area, total liquid surface area, liquid turbulence kinetic energy, dissipation rate of turbulence kinetic energy, turbulence intensity, and shear force of the culture solution in 5 types of reactor containers with different volume (CUR2.5L, CUR50L, CUR300L, CUR1200L and CUR3000L) in the working condition that the oscillator made an eccentric motion along with the vertical axis. The specific inventive steps, methods and results are as follows:

I. Building of a simulation model of the bioreactor system

1. Proposal of assumption

To make the experiment performed smoothly and control variables better, the experimental object and initial conditions were simplified before model building. Several points are assumed below:

1.1 the container of the bioreactor system is a rigid body and free of wrinkles on the surface and causes no deformation in motion;
1.2 the working environment is at standard atmospheric pressure and room temperature; and
1.3 the physical properties of the culture solution in the container are consistent with water.

2. Modeling

2.1 Geometric parameters

The structure diagram of the container of the bioreactor system is shown in FIG. 1 and the geometric parameters are listed in Table 1.

Table 1 Geometric parameters of the container of the bioreactor system

| Specification | D1 and D2 (mm) | D3 (mm) | H1 (mm) | H2 (mm) |
|---|---|---|---|---|
| CUR2.5L | 269 | 80 | 81.5 | 57 |
| CUR50L | 840 | 114 | 280 | 170 |
| CUR300L | 1500 | 190 | 422 | 303 |

(continued)

| Specification | D1 and D2 (mm) | D3 (mm) | H1 (mm) | H2 (mm) |
|---|---|---|---|---|
| CUR1200L | 1952 | 190 | 533 | 465 |
| CUR3000L | 2997 | 190 | 867 | 664 |

2.2 Rotational speed, working volume and the corresponding initial liquid level

3 rotational speed and 3 working volume (2 rotational speed were determined under the working condition of full load at CUR3000L) in each specification of the reactor were determined according to each specification of the optimal scope of rotational speed and working volume, and there were 38 sets of data in total. The specific values are shown in Table 2.

Table 2 Schematic diagram of the working condition corresponding to each specification (volume) of reactor

| Specification | Rotational speed (rpm) | Working volume (L) | Initial liquid level (m) Bottom surface of the cylinder is z=0 | Specification | Rotational speed (rpm) | Working volume (L) | Initial liquid level (m) Bottom surface of the cylinder is z=0 |
|---|---|---|---|---|---|---|---|
| CUR2.5L | 45/57/70 | 0.5 | -0.024 | CUR300L | 25/28/30 | 175 | -0.017 |
| CUR2.5L | 45/57/70 | 1.5 | 0.000 | CUR300L | 25/28/30 | 300 | 0.054 |
| CUR2.5L | 45/57/70 | 2.5 | 0.018 | CUR1200L | 20/25/30 | 150 | -0.173 |
| CUR50L | 40/50/60 | 15 | -0.05 | CUR1200L | 20/25/30 | 675 | 0.054 |
| CUR50L | 40/50/60 | 32 | -0.008 | CUR1200L | 20/25/30 | 1200 | 0.229 |
| CUR50L | 40/50/60 | 50 | 0.025 | CUR3000L | 24/26 | 3000 L | 0.189 |
| CUR300L | 25/28/30 | 50 | -0.130 | | | | |

2.3 Mesh generation

An unstructured tetrahedral mesh is used in this experiment. The mesh information is shown in Table 3.

Table 3 Information of the unstructured tetrahedral mesh in each specification of the reactor

| Specification | Maximum mesh size (m) | Node | Element | Minimum mesh mass (Min.) | Mean mesh mass (mean) |
|---|---|---|---|---|---|
| CUR2.5L | 0.016 | 4173 | 23934 | 0.3729 | 0.8407 |
| CUR50L | 0.064 | 2145 | 12149 | 0.3138 | 0.8223 |
| CUR300L | 0.128 | 1336 | 7500 | 0.4007 | 0.8073 |
| CUR1200L | 0.128 | 2803 | 15966 | 0.3562 | 0.8429 |
| CUR3000L | 0.256 | 1314 | 7334 | 0.2344 | 0.8160 |

2.4 Configuration of the initial conditions of the flow field

A 2-phase flow of a VOF model was used to trace the interface between gas and liquid; a RNG k-ε model was selected as a turbulence model. The movement locus of the bioreactor is an eccentric motion. The motion eccentricity of the CUR2.5L, CUR50L, CUR300L, CUR1200L and CUR3000L reactors was respectively 30 mm, 40 mm, 60 mm, 65 mm and 65 mm. The smoothing and layering dynamic mesh techniques were utilized in software FLUENT to compile UDF, thus defining the motion of the reactor; factors influencing the motion of the tank body were angular velocity co and eccentricity R. A kinematic equation of a certain point on the tank body is as follows:

$$V_\mathrm{x} = R\omega\cos(\omega t + \varphi_0)$$

$$V_y = -R\omega\sin(\omega t + \varphi_0)$$

where,

$V_x$ - linear velocity of the point in the x direction; $V_y$ - linear velocity of the point in the y direction;
$t$ - time; $\varphi_0$ - initial phase angle of the point (to simplify the calculation, the point is taken as an origin [0, 0, 0] during the reactor modeling).

CUR300L at a rotational speed of 25 rpm is set as an example; and the specific contents of the UDF file are as follows:

```
#include "udf.h"
#include "math.h"
#include "dynamesh_tools.h"
#define rox 0.1571
#define roy -0.1571
#define ome 2.6180
DEFINE_CG_MOTION(tank_rotation_one, dt, vel, omega,
time, dtime)
    {
        vel[0] = rox*cos(ome*time);
        vel[1] = roy*sin(ome*time);
        vel[2] = 0; }
```

II. Experimental results

1. Relative position and morphologic changes of liquid in the container under the working condition of computer simulation

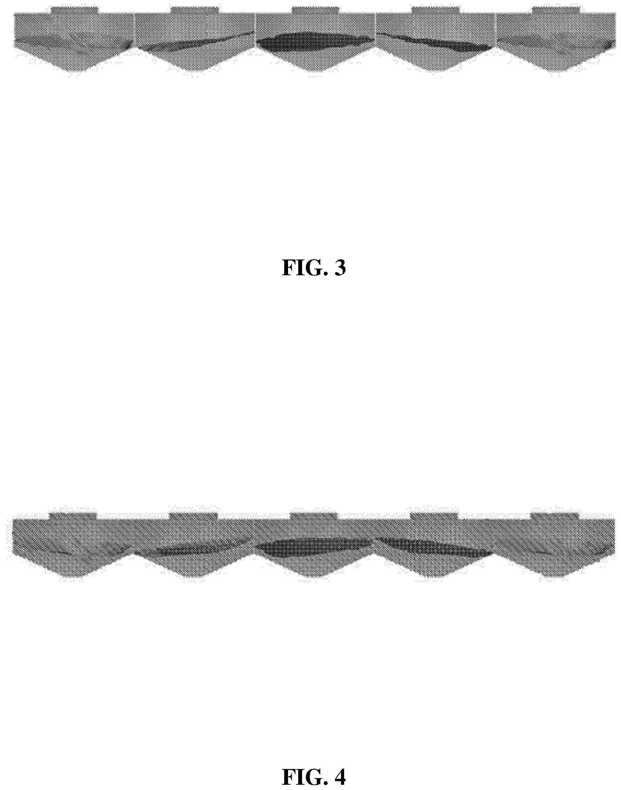

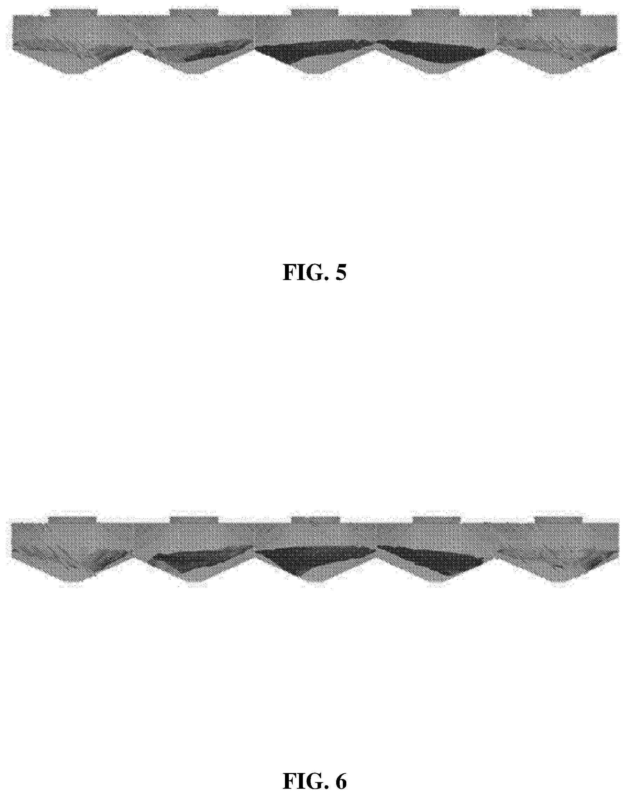

The change of liquid in the initial stage is not taken into consideration in this simulation, but the quasi-steady-state process of the culture solution in a reactor when the shape is relatively stable is surveyed only.
It can be seen from the simulated diagrams (FIGS. 3, 4, 5 and 6) on the changes of the free liquid levels when CUR300L is up to a steady state and the reactor rotates a round that the liquid is deviated from the center axis of the reactor container to different extents. At the same volume, when the rotational speed is higher, the centrifugal force on the liquid is larger, and the deviation extent is more obvious, the liquid is extruded more thinly, and the interface in contact with the air concaves more obviously. The working volume of 300 L and 175 L has the same variation trend. Meanwhile, by observing the free liquid level in the reactor during the rotation process, it can be further found that the morphologic change process of the free liquid level in the reactor is not obvious any more when being in a quasi-steady-state.

2. Total liquid surface area, gas-liquid interface area and ratio thereof to the working volume under the working condition of computer simulation

Based on the assumption of two-film theory of two-phase mass transfer, in the real process, the update rate of the gas of a liquid film adhered on an inner wall of the reactor container may be different from that of the main body. Therefore, the liquid which rotates to the position contacts the liquid film to influence the gas composition of the main body possibly. Therefore, the sum of the contact area (w) between liquid and the inner wall surface of the reactor container, and the gas-liquid interface area (A) is expressed as the total liquid surface area (S, S =W +A).

Table 4 Changes of the total liquid surface area, gas-liquid interface area and ratio thereof to the working volume along with the rotational speed in the reactor at each working volume

| Type | Working volume/L | Rotational speed (rpm) | Total liquid surface area/m$^2$ | | Gas-liquid interface area/m$^2$ | | Total liquid surface area/working volume | | Gas-liquid interface area/ Working volume | |
|---|---|---|---|---|---|---|---|---|---|---|
| | | | Static state | Dynamic state | Static state | Dynamic state | Static state | Dynamic state | Static state | Dynamic state |
| CUR2.5L | 2.5 | 60 | 0.1391 | 0.1419 | 0.0580 | 0.0589 | 55.64 | 56.76 | 23.20 | 23.56 |
| CUR2.5L | 2.5 | 70 | 0.1391 | 0.1553 | 0.0580 | 0.0618 | 55.64 | 62.12 | 23.20 | 24.72 |
| CUR5L | 5 | 55 | 0.2585 | 0.2624 | 0.1217 | 0.1237 | 51.70 | 52.48 | 24.34 | 24.74 |
| CUR5L | 5 | 65 | 0.2585 | 0.2971 | 0.1217 | 0.1281 | 51.70 | 59.42 | 24.34 | 25.62 |
| CUR50L | 50 | 40 | 1.2316 | 1.3304 | 0.5697 | 0.5796 | 24.63 | 26.61 | 11.39 | 11.59 |
| CUR50L | 50 | 55 | 1.2316 | 1.4863 | 0.5697 | 0.6385 | 24.63 | 29.73 | 11.39 | 12.77 |
| CUR500L | 500 | 28 | 5.2369 | 5.3029 | 2.2920 | 2.3103 | 10.47 | 10.61 | 4.58 | 4.62 |
| CUR500L | 500 | 30 | 5.2369 | 5.5070 | 2.2920 | 2.3341 | 10.47 | 11.01 | 4.58 | 4.67 |
| CUR1200L | 1200 | 27 | 7.8114 | 7.9005 | 3.0544 | 3.0843 | 6.51 | 6.58 | 2.55 | 2.57 |
| CUR1200L | 1200 | 29 | 7.8114 | 7.9610 | 3.0544 | 3.1061 | 6.51 | 6.63 | 2.55 | 2.59 |
| CUR3000L | 3000 | 24 | 16.7168 | 16.9434 | 7.2203 | 7.2921 | 5.57 | 5.65 | 2.41 | 2.43 |
| CUR3000L | 3000 | 26 | 16.7168 | 17.0518 | 7.2203 | 7.3522 | 5.57 | 5.68 | 2.41 | 2.45 |

It can be seen from Table 4 that the degree of influence of the increased rotational speed on S varies from the difference of the working volume. By comparison of the change trend between the total liquid surface area S and the gas-liquid interface area A in each specification of the reactor at different working volume, it is found that both keep a basically consistent change trend. When the rotational speed increases, the rotational frequency of the liquid in the reactor quickens, and the liquid swept area in unit time increases such that the update rate of the gas and liquid quickens. Therefore, the rotational speed may be simply used to roughly measure the update rate of the gas and liquid.

3. Fluid turbulence kinetic energy and the dissipation rate of turbulence kinetic energy in the reactor under the working condition of computer simulation

(1) Turbulence kinetic energy k is used to represent the energy contained in the pulsation process of fluid micelles, and the formula is as follows:

$$\mathrm{k} = \frac{3}{2}\left(v_{ave}I\right)^2$$

where,

$V_{ave}$ - mean velocity of fluid;
$I$ - turbulence intensity; and the value is equal to a ratio of a root mean square $v'$ of turbulent fluctuation velocity to mean velocity $V_{ave}$.

(2) The dissipation rate of turbulence kinetic energy $\varepsilon$ is used to measure the velocity of energy loss caused by micelle collision and viscous dissipation, and the formula is as follows:

$$\varepsilon = \rho C_\mu \frac{\mathrm{k}^2}{\mu}\left(\frac{\mu_1}{\mu}\right)^{-1}$$

where, $\rho$, $\mu$ and $\mu_1$ - density, viscosity and turbulent viscosity of a fluid.

The motion of fluid micelles may be represented as the superposition of the mean velocity thereof flowing with the main body and the fluctuation velocity of random fluctuation thereof. The random fluctuation of micelles causes turbulent flow. It can be seen from Table 5 that in case of a constant working volume, the higher the rotational speed is, the higher the tubulence energy is. Therefore, the container has better material transfer and mixing effects. Meanwhile, $\varepsilon$ also increases to different extents.

The input power increases with the increase of the rotational speed such that the proportion of the fluid micelles carrying higher energy increases and the number of microscopic eddies capable of generating effective collision for energy transmission increases, namely, manifested as the increase of the mean k. Meanwhile, the area of sweeping the inner wall of the container in unit time by the liquid increases with the increase of the rotational speed, and the opportunity of the gas-liquid contact exchange increases, and accordingly, the adhesion effect on the wall surface and viscous friction inside the liquid also increase. The increased ratio of high-energy micelles causes more micelle collision and energy transfer, and the transfer frequency of k has an accelerated attenuation rate. Therefore, the dissipation rate of turbulence kinetic energy $\varepsilon$ quickens with the increase of rotational speed.

Oxygen liquid-film transfer coefficient $k_L$ and the dissipation rate of turbulence kinetic energy $\varepsilon$ have the following relational expression:

$$\mathrm{k}_L = \frac{2}{\sqrt{\pi}}\sqrt{D_{O_2}}\left(\frac{\varepsilon\rho_L}{\mu_L}\right)^{\frac{1}{4}}$$

where, $D_{O_2}$ denotes oxygen diffusion coefficient; $\rho_L$ and $\mu_L$ respectively denote density and viscosity of a liquid phase. The above parameters are constants under this experiment conditions; the dissipation rate of turbulence kinetic energy $\varepsilon$ may be used to represent the intensity of the gas-liquid transfer process. The gas-liquid transfer becomes violent with the increase of the $\varepsilon$, and approximatively, the gas-liquid mass transfer coefficient $k_L a$ becomes larger.

Table 5 Changes of the turbulence kinetic energy k and the dissipation rate of turbulence kinetic energy $\varepsilon$ with the rotational speed in the reactor at each working volume

| Specificatio n | Working volume (L) | Rotational speed (rpm) | Turbulence kinetic energy k | Dissipation rate of turbulence kinetic energy $\varepsilon$ |
|---|---|---|---|---|
| CUR2.5L | 0.5 | 45 | 3.88E-04 | 0.0008 |
| | 0.5 | 57 | 1.50E-03 | 0.0073 |
| | 0.5 | 70 | 3.46E-03 | 0.0266 |
| | 1.5 | 45 | 7.18E-04 | 0.0024 |
| | 1.5 | 57 | 1.43E-03 | 0.0074 |
| | 1.5 | 70 | 3.00E-03 | 0.0241 |
| | 2.5 | 45 | 4.08E-04 | 0.0008 |
| | 2.5 | 57 | 1.33E-03 | 0.0081 |
| | 2.5 | 70 | 2.38E-03 | 0.0213 |
| CUR50L | 15 | 40 | 7.65E-03 | 0.0197 |
| | 15 | 50 | 9.78E-03 | 0.0326 |
| | 15 | 60 | 1.24E-02 | 0.0634 |
| | 32 | 40 | 5.41E-03 | 0.0173 |
| | 32 | 50 | 8.45E-03 | 0.0310 |
| | 32 | 60 | 1.04E-02 | 0.0599 |
| | 50 | 40 | 4.67E-03 | 0.0142 |
| | 50 | 50 | 7.56E-03 | 0.0200 |
| | 50 | 60 | 8.37E-03 | 0.0489 |
| CUR300L | 50 | 25 | 7.52E-03 | 0.0103 |
| | 50 | 28 | 9.64E-03 | 0.0159 |
| | 50 | 30 | 1.29E-02 | 0.0211 |
| | 175 | 25 | 5.03E-03 | 0.0088 |
| | 175 | 28 | 8.06E-03 | 0.0156 |
| | 175 | 30 | 9.95E-03 | 0.0204 |
| | 300 | 25 | 3.50E-03 | 0.0049 |
| | 300 | 28 | 7.15E-03 | 0.0150 |
| | 300 | 30 | 9.01E-03 | 0.0194 |
| CUR1200L | 150 | 20 | 6.58E-03 | 0.0102 |
| | 150 | 25 | 9.20E-03 | 0.0203 |
| | 150 | 30 | 1.63E-02 | 0.0391 |
| | 675 | 20 | 5.34E-03 | 0.0101 |
| | 675 | 25 | 8.73E-03 | 0.0197 |
| | 675 | 30 | 1.42E-02 | 0.0313 |
| | 1200 | 20 | 5.00E-03 | 0.0092 |
| | 1200 | 25 | 9.30E-03 | 0.0151 |
| | 1200 | 30 | 9.94E-03 | 0.0205 |
| CUR3000L | 3000 | 24 | 2.48E-02 | 0.0477 |
| | 3000 | 26 | 3.36E-02 | 0.0817 |

4. Turbulence intensity in the reactor under the working condition of computer simulation

Turbulent diffusion plays a leading role from the angle of micro-environment. The motion of fluid micelles may be represented as the superposition of the mean velocity thereof flowing with the main body and the fluctuation velocity of random fluctuation thereof. The random fluctuation of micelles causes turbulent diffusion. The stronger the interaction between micelles is, the stronger the interaction is, and the more significant the energy transfer and dispersion effect are. Therefore, the stronger the turbulence intensity is, the better the material transfer and mixing effects within a liquid phase are.

$I$ may be used to represent the intensity of the velocity fluctuation and interaction of the fluid micelles. There exists violent velocity fluctuation among the micelles; the stronger the interaction is, the more significant the energy transfer and dispersion effect are. Therefore, the stronger the turbulence intensity is, the better the material transfer and mixing effects within a liquid phase are. The formula of turbulence intensity $I$ is as follows:

$$I = \frac{v'}{v_{avg}}$$

where, $v'$ is a root mean square of turbulent fluctuation velocity; $v_{avg}$ is mean velocity of fluid. The motion of fluid micelles may be represented as the superposition of the mean velocity thereof flowing with the main body and the fluctuation velocity of random fluctuation thereof. The random fluctuation of micelles causes turbulent flow.

It can be seen from Table 6 that in case of a constant working volume, with the increase of the rotational speed, $I$ shows an increased trend; the improved rotational speed increases the energy input to fluid, thus resulting in increased kinetic energy of fluid micelles, accelerated fluctuation velocity and correspondingly increased turbulence intensity.

Table 6 Changes of the turbulence intensity I with the rotational speed in the reactor at each working volume

| Specification | Working volume (L) | Rotational speed (rpm) | Turbulence intensity I (%) | Specification | Working volume (L) | Rotational speed (rpm) | Turbulence intensity I(%) |
|---|---|---|---|---|---|---|---|
| CUR2.5L | 0.5 | 45 | 2.16 | CUR50L | 15 | 40 | 6.76 |
| | 0.5 | 57 | 3.00 | | 15 | 50 | 7.65 |
| | 0.5 | 70 | 4.62 | | 15 | 60 | 9.46 |
| | 1.5 | 45 | 1.99 | | 32 | 40 | 5.67 |
| | 1.5 | 57 | 2.83 | | 32 | 50 | 7.19 |
| | 1.5 | 70 | 4.24 | | 32 | 60 | 8.91 |
| | 2.5 | 45 | 1.51 | | 50 | 40 | 3.00 |
| | 2.5 | 57 | 2.61 | | 50 | 50 | 6.21 |
| | 2.5 | 70 | 3.63 | | 50 | 60 | 7.61 |
| CUR300L | 50 | 25 | 5.46 | CUR1200L | 150 | 20 | 4.14 |
| | 50 | 28 | 7.43 | | 150 | 25 | 5.62 |
| | 50 | 30 | 9.06 | | 150 | 30 | 10.07 |
| | 175 | 25 | 5.38 | | 675 | 20 | 5.42 |
| | 175 | 28 | 7.00 | | 675 | 25 | 6.92 |
| | 175 | 30 | 7.85 | | 675 | 30 | 9.30 |
| | 300 | 25 | 4.55 | | 1200 | 20 | 4.43 |
| | 300 | 28 | 6.35 | | 1200 | 25 | 5.48 |
| | 300 | 30 | 7.20 | | 1200 | 30 | 8.67 |
| CUR3000L | 3000 | 24 | 11.56 | | | | |
| | 3000 | 26 | 13.12 | | | | |

5. Mean shear rate

Animal cells have no cell walls outside the cytomembrane and thus, are more sensitive to the dynamic environment within a flow field. The mechanical damage caused by an external force on cells is a non-negligible factor in the design and use processes of the animal cell bioreactor.

The shear force on cells in the bioreactor is divided into major three categories: a flow field shear force generated by the relative motion inside a culture solution; and the flow field shear force is in direct proportion to a velocity gradient and a kinematic viscosity of the culture solution; a shear force generated when bubbles rise in a culture solution and the surface is broken; and a shear force generated by the collision between cells and the inner wall of a reactor as well as the mutual collision between cells. The damage of the flow field shear force on cells is closely related to the minimum eddy size generated by fluid turbulence. The culture solution is adhered on a wall surface of a container to rotate, thus producing a turbulent eddy; and the large eddy gradually develops into a small eddy, and then the small eddy develops into a smaller eddy; and the process is accompanied with energy transfer, namely, the mechanical energy of the culture solution changes to heat energy, thus resulting in the increase of temperature. During the gradient change of the eddy, when the size of the eddy is close to the size of cells, energy is transferred to the cells by the eddy, thus resulting in cell rupture. For the bioreactor of the present application, because a surface oxygen transfer mode is used and there is no bubble sparger, there is precious little bubble generated. Accordingly, the shear force damage caused by bubbles may be ignored. Because the wall surfaces inside a tank body of the bioreactor are relatively smooth, the collision shear force is also relatively weak. Through the above analysis, it can be seen that the shear force in a torrent bioreactor mainly refers to a flow field shear force. Therefore, through the simulation test, the mean shear force inside the reactor has important reference values to the design of the bioreactor.

Table 7 Changes of the mean shear force with the rotational speed in the reactor at each working volume

| Specification | Working volume (L) | Rotational speed (rpm) | Mean shear rate (s^-1) | Specification | Working volume (L) | Rotational speed (rpm) | Mean shear rate (s^-1) |
|---|---|---|---|---|---|---|---|
| CUR2.5L | 0.5 | 45 | 4.9853 | CUR50L | 15 | 40 | 7.9708 |
| | 0.5 | 57 | 10.1582 | | 15 | 50 | 8.9876 |
| | 0.5 | 70 | 20.2738 | | 15 | 60 | 9.3835 |
| | 1.5 | 45 | 6.2612 | | 32 | 40 | 7.2709 |
| | 1.5 | 57 | 10.9229 | | 32 | 50 | 8.7364 |
| | 1.5 | 70 | 19.8577 | | 32 | 60 | 9.0551 |
| | 2.5 | 45 | 4.0946 | | 50 | 40 | 6.9012 |
| | 2.5 | 57 | 10.8802 | | 50 | 50 | 8.2140 |
| | 2.5 | 70 | 17.8340 | | 50 | 60 | 8.9004 |
| CUR300L | 50 | 25 | 4.2551 | CUR1200L | 150 | 20 | 3.8602 |
| | 50 | 28 | 5.9395 | | 150 | 25 | 6.2380 |
| | 50 | 30 | 6.7627 | | 150 | 30 | 6.2714 |
| | 175 | 25 | 3.9559 | | 675 | 20 | 3.2128 |
| | 175 | 28 | 5.2161 | | 675 | 25 | 5.4725 |
| | 175 | 30 | 5.7067 | | 675 | 30 | 5.8422 |
| | 300 | 25 | 3.3894 | | 1200 | 20 | 1.3091 |
| | 300 | 28 | 4.8398 | | 1200 | 25 | 4.4204 |
| | 300 | 30 | 5.3338 | | 1200 | 30 | 4.9002 |
| CUR3000L | 3000 | 24 | 3.9816 | | | | |
| | 3000 | 26 | 4.4238 | | | | |

It can be obtained from Table 7 that the mean value of the flow field shear rate of the culture solution in a steady state of motion is 20.27/s below.

The mean shear rate refers that the shear rates in the whole flow field region are subjected to volume integral, and then averaging. The calculation formula is as follows:

$$\overline{\tau} = \frac{1}{\sum\limits_{i-1}^{n} V_i} \sum\limits_{i-1}^{n} V_i \cdot V_i$$

It can be obtained from Table 7 that for CUR2.5L, in case of a constant working volume, the mean shear rate increases with the increase of the rotational speed of the oscillator, and the change of the working volume causes relatively low influences on the mean shear rate. The mean shear rate on CUR1200L is much smaller than that of CUR2.5L. When the mean shear rate decreases, damage on the cells also reduces, but the shear rate and the mixing effect are mutually constrained; the decrease of the shear rate means that the mixing effect becomes poor.

Chinese hamster ovary cells (CHO) were set as an example; when the shear force was greater than 0.392 Pa, namely, the shear rate in water was greater than $391.41 s^{-1}$, the cells would be damaged. The mean shear rate in the reactor obtained from Table 7 is much lower than the shear rate when the cells are damaged.

III. Brief summary

CUR2.5L, CUR50L, CUR300L, CUR1200L and CUR3000L served as simulation objects in this experiment. A dynamic mesh model, a RNG k $\varepsilon$ turbulence model and in combination with a VOF model were used to respectively perform numerical analysis on the flow behaviors of liquid in a container at 14 rotational speed and 13 working volume (38 working conditions in total). The results indicate that:

1. the shape of the liquid phase is obviously influenced when being up to the quasi-steady-state, and the bending degree of the liquid level aggravates with the increase of rotational speed;
2. the gas-liquid interface area $A$ will not increase linearly with the increase of the rotational speed; the degree of influence of the increased rotational speed on the total liquid surface area $S$ varies from the difference of the working volume;
3. rated turbulence parameters of a liquid phase will be dually influenced by the rotational speed and working volume; according to the relational expression of $k_L$ and $\varepsilon$, $\varepsilon$ may be used to approximatively represent the intensity of the gas-liquid transfer process;
4. the mean shear rate increases with the increase of the rotational speed. When the mean shear rate decreases, damage on the cells also reduces, but the shear rate and the mixing effect are mutually constrained; the decrease of the shear rate means that the mixing effect becomes poor. The mean shear rate in the reactor is much lower than the shear rate when the cells are damaged.

**Example 2 Test on the mixing properties of the bioreactor system**

I. Test on the mixing properties of a CUR5L bioreactor

1. Experimental design

Cell culture environment of the bioreactor was simulated to determine the mixing unevenness of materials in the bioreactor at different working volume and rotational speed, thus verifying the mixing properties of the reactor.
Equipment type: CUR5L bioreactor (JYSS, D1=400 mm, D3=80 mm, H1=149 mm and H2=98 mm) with working volume of 5 L; the solution for mixing is a PBS buffer solution, and the reagent added is 1 mol/L NaOH. Detecting instrument: Mettler pH electrodes.
Determination contents: after a NaOH reagent was added to the bioreactor, the time of mixing uniformly was determined by pH electrodes, thus determining the time of mixing the materials uniformly added to the container during the oscillating process of the reactor. The duration of time served to simulate the ability of the added materials to influencing cell culture actually in the practical use process of the bioreactor.
The conditions of normal cell culture of a reactor were simulated in this experiment to respectively configure three rotational speed of 50 rpm, 55 rpm and 60 rpm and three working volume (minimum working volume of 2 L, optimal working volume of 3.5 L and maximum working volume of 5 L).

2. Experiment preparation

(1) A 15 L PBS buffer solution was prepared for further use in the environment conforming to the GMP standards.
(2) 500 mL NaOH solution having a concentration of 1 mol/L was prepared as a standard experiment solution in the environment conforming to the GMP standards; afterwards, the solution was sealed in a 500 mL solution storage barrel to prevent the spoil of the solution, influencing the accuracy of the experiment.
(3) A pH sensor was put on an interface at the top of the reactor to reflect the mixing efficiency by measuring the pH of the solution.

3. Experiment steps

3.1 Mixing experiments of the bioreactor CUR5L at the working volume of 2 L and rotational speed of 50, 55 and 60 rpm

(1) 2 L PBS buffer solution was first added to the reactor; the temperature was controlled within 25-27 °C and the rotational speed of the reactor was set 50 rpm; the buffer solution made an oscillatory motion by an oscillator such that the motion eccentricity of the reactor was 30 mm.
(2) After the reactor got into the steady-state motion, 3 mL NaOH solution having a concentration of 1 mol/L was injected into the reactor from the center of a circle right above the reactor. The NaOH solution was injected once every 5 minutes for three times in total.
(3) The rotational speed of the reactor was set 55 rpm, and the step (2) was repeated.
(4) The rotational speed of the reactor was set 60 rpm, and the step (2) was repeated.

3.2 Mixing experiments of the bioreactor CUR5L at the working volume of 3.5 L and rotational speed of 50, 55 and 60 rpm

The steps were basically the same as 3.1 except the volume of the PBS buffer solution added to the reactor was 3.5 L and the volume of the NaOH solution having a concentration of 1 mol/L injected into the reactor every time was 5 mL.

3.3 Mixing experiments of the bioreactor CUR5L at a working volume of 5 L and rotational speed of 50, 55 and 60 rpm

The steps were basically the same as 3.1 except the volume of the PBS buffer solution added to the reactor was 5 L and the volume of the NaOH solution having a concentration of 1 mol/L injected into the reactor every time was 10 mL.
pH sensors were used to real-timely monitor the change of pH in the bioreactor, the used time of reaching the same pH of the two sensors was observed; image data and report data of the sensors were exported to analyze the used time of mixing evenly detected by the two sets of sensors, thus obtaining the mixing efficiency of the reactor.

4. Experimental results

Standard for the stable pH of the solution: when a pH value is stabilized at a certain value for consecutive 10 s, and the error range is within ±0.01, the pH value is considered to be stable. The time used to be a stable pH value at each condition is summarized in Table 8 below.

Table 8 Time to achieving a stable pH value of the CUR5L at different working volume and rotational speed

| Working volume (L) Mixing time (s) Rotational speed (rpm) | 2 L (mm) | 3.5 L | 5 L |
| --- | --- | --- | --- |
| 50 rpm | 19 | 16 | 25 |
| 55 rpm | 20 | 14 | 19 |
| 60 rpm | 14 | 15 | 13 |

II. Test on the mixing properties of a CUR50L bioreactor

A CUR50L bioreactor (JYSS, D1=840 mm, D3=114 mm, H1= 280 mm and H2=170 mm) was used; three rotational speed of 38 rpm, 39 rpm and 40 rpm and three working volume (minimum working volume of 15 L, optimal working volume of 30 L and maximum working volume of 50 L) were respectively set, and eccentricity was set 40 mm, and the temperature was controlled within 25-27 °C.
The test method was basically the same as the above. A 15 L PBS buffer solution was added to the reactor and a 10 mL NaOH solution having a concentration of 1 mol/L was injected into the reactor every time at the working volume of 15 L. A 30 L PBS buffer solution was added to the reactor and a 30 mL NaOH solution having a concentration of 1 mol/L was injected into the reactor every time at the working volume of 30 L. A 50 L PBS buffer solution was added to the reactor and a 50 mL NaOH solution having a concentration of 1 mL was injected into the reactor every time at the working volume of 50 L. The results are summarized in Table 9.

Table 9 Time to achieving a stable pH value of the CUR50L at different working volume and rotational speed

| Working volume (L) Mixing time (s) Rotational speed (rpm) | 15 L | 30 L | 50 L |
|---|---|---|---|
| 38 rpm | 50 | 52 | 72 |
| 39 rpm | 35 | 34 | 142 |
| 40 rpm | 32 | 41 | 101 |

III. Test on the mixing properties of a CUR500L bioreactor

A CUR500L bioreactor (JYSS, D1=1690 mm, D3=190 mm, H1=452 mm and H2=347 mm) was used; three rotational speed of 25 rpm, 27 rpm and 29 rpm and three working volume (minimum working volume of 100 L, optimal working volume of 300 L and maximum working volume of 500 L) were respectively set, and eccentricity was set 65 mm, and the temperature was controlled within 25-27 °C.

The test method was basically the same as the above. A 100 L PBS buffer solution was added to the reactor and a 100 mL NaOH solution having a concentration of 1 mL was injected into the reactor every time at the working volume of 50 L. A 300 L PBS buffer solution was added to the reactor and a 100 mL NaOH solution having a concentration of 1 mL was injected into the reactor every time at the working volume of 300 L. A 500 L PBS buffer solution was added to the reactor and a 100 mL NaOH solution having a concentration of 1 mL was injected into the reactor every time at the working volume of 500 L. The results are summarized in Table 10.

Table 10 Time to achieving a stable pH value of the CUR500L at different working volume and rotational speed

| Working volume (L) Mixing time (s) Rotational speed (rpm) | 100 L | 300 L | 500 L |
|---|---|---|---|
| 25 rpm | 86 | 82 | 90 |
| 27 rpm | 97 | 62 | 80 |
| 29 rpm | 40 | 94 | 70 |

IV. Test on the mixing properties of a CUR1200L bioreactor

A CUR1200L (mm) bioreactor (JYSS, D1=1952 mm, D3=190 mm, H1=533 mm and H2=465 mm) was used; three rotational speed of 23 rpm, 25 rpm and 27 rpm and three working volume (minimum working volume of 300 L, optimal working volume of 800 L and maximum working volume of 1200 L) were respectively set, and eccentricity was set 65 mm, and the temperature was controlled within 25-27 °C.

The test method was basically the same as the above. A 300 L PBS buffer solution was added to the reactor and a 100 mL NaOH solution having a concentration of 1 mL was injected into the reactor every time at the working volume of 300 L. A 800 L PBS buffer solution was added to the reactor and a 800 mL NaOH solution having a concentration of 1 mol/L was injected into the reactor every time at the working volume of 800 L. A 1200 L PBS buffer solution was added to the reactor and a 500 mL NaOH solution having a concentration of 2 mol/L was injected into the reactor every time at the working volume of 1200 L. The results are summarized in Table 11.

Table 11 Time to achieving a stable pH value of the CUR1200L at different working volume and rotational speed

| Working volume (L) Mixing time (s) Rotational speed (rpm) | 300 L | 800 L | 1200 L |
|---|---|---|---|
| 23 rpm | 56 | 110 | 679 |
| 25 rpm | 49 | 253 | 174 |
| 27 rpm | 55 | 257 | 72 |

As shown in the data of Tables 8-11, the mixing efficiency of the device may completely satisfy the mixing and mass transfer requirements of the large-scale cell culture process.

**Example 3 MDCK cell culture**

The CUR5L, CUR50L, CUR500L and CUR1200L used in Example 2 were used for MDCK cell culture. The cell inoculum density was $1.5 \times 10^6$ cell/ml; the growth medium was CD MDCK SFM (DP304, JSBio); the culture temperature was 37°C and the dissolved oxygen value DO% was 35-65.

The CUR5L reactor was configured with a working volume of 5 L, a rotational speed of 55 rpm and an eccentricity of 30 mm; the importing rate of oxygen and air was respectively set 100 mL/min and 200 mL/min. The CUR50L reactor was configured with a working volume of 50 L, a rotational speed of 40 rpm and an eccentricity of 40 mm; the importing rate of oxygen and air was respectively set 500 mL/min and 500 mL/min. The CUR500L reactor was configured with a working volume of 500 L, a rotational speed of 28 rpm and an eccentricity of 65 mm; the importing rate of oxygen and air was respectively set 900 mL/min and 1000 mL/min. The CUR1200L reactor was configured with a working volume of 1200 L, a rotational speed of 25 rpm and an eccentricity of 65 mm; the importing rate of oxygen and air was respectively set 1500 mL/min and 1500 mL/min.

When the cells were cultured for 48 h, 2 g/L glucose was replenished for once to the reaction system, and the replenished glucose solution had a concentration of 200 g/L. Furthermore, the pH value of the bioreactor was set within a scope of 7.0-7.4; when the pH value was detected to be lower than 7.0 by the system, the system would automatically replenish $NaHCO_3$ having a concentration of 7.5%.

The cell culture data is summarized in Table 12 below.

Table 12 MDCK cell culture data

| Specification | Culture time (h) | Initial volume of the medium (L) | Cell density ($\times 10^6$/mL) | Cell viability (%) | Total oxygen consumption (L) |
|---|---|---|---|---|---|
| CUR5L | 0 | 5 L | 1.50 | 99.2 | 4.7 |
| | 24 | | 3.00 | 99.5 | |
| | 48 | | 5.30 | 99.2 | |
| | 72 | | 8.20 | 99.4 | |
| CUR50L | 0 | 50 L | 1.50 | 99.4 | 49 |
| | 24 | | 2.90 | 99.3 | |
| | 48 | | 6.00 | 99.5 | |
| | 72 | | 7.80 | 99.6 | |
| CUR500L | 0 | 500 L | 1.50 | 99.4 | 438 |
| | 24 | | 2.40 | 99.2 | |
| | 48 | | 4.80 | 99.8 | |
| | 72 | | 7.47 | 99.6 | |
| CUR1200L | 0 | 1200 L | 1.50 | 99.5 | 2850 |
| | 24 | | 2.30 | 99.2 | |
| | 48 | | 5.20 | 99.6 | |
| | 72 | | 7.30 | 99.4 | |

It can be seen from Table 12 that in the cell culture process, even if the cell density increases constantly, the cell viability is always kept above 99.0%.

**Example 4 CHO cell culture**

1. CHO cells cultured in a CUR300L bioreactor

The CUR300 L bioreactor (JYSS, D1=1500 mm, D3=190 mm, H1=422 mm and H2=303 mm) constructed in this present application was used, and a Fed-batch cell culture process was taken to culture the CHO cells. The reactor had a rotational speed of 30 rpm and an eccentricity of 60 mm; the initial volume of media was respectively 200 L, 205 L and 250 L; the temperature was controlled at 37°C; the importing rate of oxygen and air was respectively set 800 mL/min and 800 mL/min. The initial medium is a dedicated medium for CHO cells from Gansu JSBio; and on the 4th day of the culture, the replenished medium is CD Feed002 from Gansu JSBio.

The inoculum density of the CHO cells was about $1.0 \times 10^6$/mL; CD Feed002 which were 5%, 2%, 5% and 2% of the current working volume on the 4th, 6th, 8th and 10th days were respectively added; when glucose was lower than 2.0 g/L, a glucose solution was replenished to 8.0 g/L; the pH value was set within a scope of 7.0-7.2; when the pH value was lower than 7.0, the system would automatically replenish NaHCO$_3$ having a concentration of 7.5%. The culture was continued for 14 d; the density, viability, osmotic pressure, oxygen partial pressure, partial pressure of carbon dioxide, protein expression quantity and other conditions of cell growth were observed every day. The cell density and viability during the culture process are recorded in Table 13.

Table 13 CHO cell culture data

| Culture time (h) | Initial volume of the medium (L) | Cell density ($\times 10^6$ /mL) | Cell viability (%) | Initial volume of the medium (L) | Cell density ($\times 10^6$ /mL) | Cell viability (%) | Initial volume of the medium (L) | Cell density ($\times 10^6$ /mL) | Cell viability (%) |
|---|---|---|---|---|---|---|---|---|---|
| 0 | | 0.99 | 95.9 | | 0.96 | 97.8 | | 1.19 | 98.7 |
| 24 | | 1.36 | 96.3 | | 1.57 | 98.7 | | 2.13 | 98.8 |
| 48 | | 3.97 | 97.0 | | 3.22 | 98.5 | | 4.28 | 99.2 |
| 72 | | 6.82 | 98.1 | | 5.94 | 99.1 | | 7.75 | 98.8 |
| 96 | | 10.70 | 98.8 | | 9.00 | 97.8 | | 13.07 | 99.2 |
| 120 | | 14.70 | 97.2 | | 13.90 | 98.6 | | 16.45 | 98.9 |
| 144 | | 15.70 | 97.7 | | 14.60 | 98.7 | | 19.12 | 98.9 |
| 168 | 200 L | 14.30 | 97.1 | 205 L | 14.00 | 97.7 | 250 L | 19.09 | 98.1 |
| 192 | | 14.50 | 95.6 | | 13.70 | 98.1 | | 18.58 | 97.5 |
| 216 | | 13.50 | 96.8 | | 13.20 | 98.2 | | 16.77 | 96.8 |
| 240 | | 13.30 | 95.3 | | 12.50 | 98.5 | | 15.17 | 94.0 |
| 264 | | 11.20 | 94.2 | | 12.00 | 97.5 | | 16.02 | 96.5 |
| 288 | | 11.60 | 95.7 | | 11.60 | 98.3 | | 14.77 | 91.5 |
| 312 | | 11.40 | 93.3 | | 11.50 | 96.1 | | 14.73 | 96.0 |
| 336 | | 11.20 | 96.6 | | 10.80 | 96.4 | | 15.49 | 96.0 |

The culture volume when a tank was placed for harvesting on the 14th day was respectively up to 247 L, 256 L and 312 L; and the final protein expression quantity was up to 1.133 g/L, 1.594 g/L and 1.311 g/L. Throughout the culture process, the maximum cell density is up to $15.49 \times 10^6$ cell/mL; and the cells always maintain a high livability and keep a high expression periods of protein, bringing more ideal results. 2. CHO cells cultured in a CUR1200L bioreactor

The CUR1200 L bioreactor (JYSS, D1=1952 mm, D3=190 mm, H1=533 mm and H2=465 mm) was used, and a Fed-batch way was taken to culture the CHO cells. The rotational speed of the reactor was set at 30 rpm; the working volume was respectively set 250 L and 330 L; the eccentricity was set 65 mm; and the temperature was controlled at 37°C. The importing rate of oxygen and air was respectively set 1500 mL/min and 1500 mL/min. The initial medium is a dedicated medium for CHO cells from Gansu JSBio; and on the 4th day of the culture, the replenished medium is CD Feed002 from Gansu JSBio.

The inoculum density was about $1.0 \times 10^6$ /mL; CD Feed002 which were 5%, 2%, 5% and 2% of the current working volume on the 4th, 6th, 8th and 10th days were respectively added; when glucose was lower than 2.0 g/L, a glucose solution was replenished to 8.0 g/L; the pH value was set within a scope of 7.0-7.2; when the pH value was lower than 7.0, the system would automatically replenish NaHCO$_3$ having a concentration of 7.5%. The cell culture was continued for 14 d; the density, viability, osmotic pressure, oxygen partial pressure, partial pressure of carbon dioxide, protein expression quantity and other conditions of cell growth were observed every day. The cell density and viability during the culture process are recorded in Table 14.

The culture volume when a tank was placed for harvesting on the 14th day was respectively up to 314 L and 417 L; and the final protein expression quantity was up to 1.481 g/L, and 1.308 g/L. Throughout the culture process, the maximum cell density is up to $18.51 \times 10^6$ cell/mL; and the cells always maintain a high livability and keep a high expression periods of protein, bringing more ideal results.

Table 14 CHO cell culture data

| Culture time (h) | Initial volume of the medium (L) | Cell density ($\times 10^6$ /mL) | Cell viability (%) | Initial volume of the medium (L) | Cell density ($\times 10^6$ /mL) | Cell viability (%) |
|---|---|---|---|---|---|---|
| 0 | | 1.09 | 99.1 | | 1.19 | 99.2 |
| 24 | | 2.11 | 99.0 | | 2.36 | 98.6 |
| 48 | | 5.51 | 98.6 | | 4.90 | 98.9 |
| 72 | | 9.12 | 98.4 | | 9.22 | 98.9 |
| 96 | | 15.25 | 98.7 | | 14.38 | 98.9 |
| 120 | | 19.71 | 98.2 | | 20.45 | 99.2 |
| 144 | | 20.01 | 97.4 | | 23.38 | 98.9 |
| 168 | 250 L | 18.47 | 95.0 | 330 L | 24.37 | 97.9 |
| 192 | | 17.26 | 93.5 | | 21.49 | 96.3 |
| 216 | | 18.45 | 96.2 | | 20.02 | 95.5 |
| 240 | | 17.42 | 92.8 | | 18.14 | 94.6 |
| 264 | | 17.32 | 96.1 | | 19.81 | 94.4 |
| 288 | | 16.50 | 93.0 | | 18.22 | 94.7 |
| 312 | | 16.83 | 95.3 | | 18.47 | 94.7 |
| 336 | | 15.40 | 91.3 | | 18.51 | 93.6 |

3. CHO cells cultured in a CUR50L bioreactor

The CUR50L (mm) bioreactor (JYSS, D1=1500 mm, D3=190 mm, H1=422 mm and H2=303 mm) constructed in this present application was used, and an ATF Perfusion cell culture process was taken to culture the CHO cells. The rotational speed of the reactor was set at 37 rpm (the 1st day to the 8th day) and 39 rpm (the 9th day to the 20th day); the working volume was 18 L; the eccentricity was 40 mm; and the temperature was controlled at 37°C. The importing rate of oxygen and air was respectively set 500 mL/min and 500 mL/min. The initial medium is a dedicated medium for CHO cells from Gansu JSBio; and on the 11th day of the culture, the replenished medium is CD Feed from Gansu JSBio.

Table 15 CHO cell culture data

| Culture time (h) | Volume removed/added (L) | Cell density ($\times 10^6$ /mL) | Cell viability (%) | Culture time (h) | Volume removed/added (L) | Cell density ($\times 10^6$/mL) | Cell viability (%) |
|---|---|---|---|---|---|---|---|
| 0 | 0 | 1.1 | 97.8 | 264 | 54 | 70.9 | 99.2 |
| 24 | 0 | 1.4 | 97.4 | 288 | 54 | 84.9 | 99.1 |
| 48 | 54 | 2.4 | 98.6 | 312 | 54 | 84.6 | 98.3 |
| 72 | 54 | 3.7 | 98.3 | 336 | 54 | 86.7 | 98.6 |
| 96 | 54 | 5.5 | 98.1 | 360 | 54 | 88.5 | 98.7 |
| 120 | 54 | 8.2 | 98.4 | 384 | 54 | 89.4 | 98.7 |
| 144 | 54 | 12.6 | 98.9 | 408 | 54 | 90.1 | 98.9 |
| 168 | 54 | 18.1 | 98.6 | 432 | 54 | 82.7 | 98.7 |
| 192 | 54 | 34.3 | 98.8 | 456 | 54 | 76.8 | 98.6 |

(continued)

| Culture time (h) | Volume removed/added (L) | Cell density ($\times 10^6$ /mL) | Cell viability (%) | Culture time (h) | Volume removed/added (L) | Celldensity ($\times 10^6$/mL) | Cell viability (%) |
|---|---|---|---|---|---|---|---|
| 216 | 54 | 43.7 | 99.2 | 480 | | 72.2 | 98.6 |
| 240 | 54 | 56.6 | 99.0 | | | | |

The inoculum density was about $1 \times 10^6$/mL; a portion of culture solution was taken out through ATF-4 on the 12th day to the 20th day, and the equal volume of culture solution was added; the glucose concentration in the culture system was kept not lower than 3.0 g/L, and the pH value was set within a scope of 7.0-7.2; when the pH value was lower than 7.0, the system would automatically replenish $NaHCO_3$ having a concentration of 7.5%. The cell culture was continued for 20 d; the density, viability, diameter, osmotic pressure, residual volume of lactic acid, protein expression quantity and other conditions of cell growth were observed every day. The cell density and viability during the culture process are recorded in Table 15.

The culture volume when a tank was placed for harvesting on the 20th day was 14 L; and the final protein expression quantity was up to 27 g/L. The result exceeds the expected value. Throughout the culture process, the maximum cell density is up to $90.1 \times 10^6$ cell/mL; and the cells always maintain a high livability (97% above).

It can be seen from the above description that when the bioreactor constructed by the present application is used, and proper rotational speed, eccentricity and the like are selected, the cells are cultured smoothly, and the cell density increases gradually, and the cell viability is always kept at a higher level, for example, above 90.0%. Thus, as can be seen, the bioreactor system of the present application is suitable for high-density animal cell culture.

**Claims**

1.  A bioreactor system for culturing cells without cell walls, comprising:

    a container, comprising a hollow cylinder with a diameter of D1 and a height of H1, and a hollow circular truncated cone with an upper diameter of D2, a lower diameter of D3, and a height of H2, wherein the hollow cylinder is connected to a top surface of the hollow circular truncated cone, and D1 = D2;
    an oscillator, configured to cause the container to make an eccentric motion according to a certain eccentricity and rotational speed;
    a ventilation device, configured to introduce an oxygen-containing gas from an upper portion of the container to the inside of the container, and
    a culture solution filled in the container, of which a top surface is exposed to the oxygen-containing gas;

    wherein the oscillator is configured to maintain the eccentric motion of the container, such that a ratio of the total liquid surface area to the volume (S/V) of the culture solution in a steady state of motion is 5.65 or more, a turbulence kinetic energy is 2.73E-03 $m^2/s^2$ or more, and a flow field shear rate is 20.27/s or less, wherein the total liquid surface area is the sum of the contact area between the culture solution and the reactor wall surface and the contact area between the top surface and the gas.

2.  The bioreactor system of claim 1, wherein CFD simulation is achieved by FLUENT software.

3.  The bioreactor system of claim 1, further comprising a disposable culture bag which is disposed in the container and used for holding the culture solution, and has a shape corresponding to the container when expanded.

4.  The bioreactor system of claim 3, wherein the disposable culture bag comprises a multifunctional cover plate, and the multifunctional cover plate is provided with a plurality of connecting holes leading to the inside of the disposable culture bag.

5.  The bioreactor system of claim 1, wherein the container has the D1 and the D2 of 400-2997 mm, the D3 of 80-190 mm, the H1 of 149-867 mm, the H2 of 98-664 mm, and contains 5-3000 L cell culture solution; and the oscillator has the rotational speed of 55-24 rpm and the eccentricity of 30-65 mm.

6. The bioreactor system of claim 1, wherein the container has the D1 and the D2 of about 400 mm, the D3 of about 80 mm, the H1 of about 149 mm, the H2 of about 98 mm, and contains about 5 L cell culture solution; and the oscillator has the rotational speed of about 55 rpm and the eccentricity of about 30 mm; wherein "about" refers to a range of the value ±20% .

7. The bioreactor system of claim 1, wherein the container has the D1 and the D2 of about 840 mm, the D3 of about 114 mm, the H1 of about 280 mm, the H2 of about 170 mm, and contains about 50 L cell culture solution; and the oscillator has the rotational speed of about 40 rpm and the eccentricity of about 40 mm; wherein "about" refers to a range of the value ±20% .

8. The bioreactor system of claim 1, wherein the container has the D1 and the D2 of about 1690 mm, the D3 of about 190 mm, the H1 of about 452 mm, the H2 of about 347 mm, and contains about 500 L cell culture solution; and the oscillator has the rotational speed of about 28 rpm and the eccentricity of about 65 mm; wherein "about" refers to a range of the value ±20% .

9. The bioreactor system of claim 1, wherein the container has the D1 and the D2 of about 1952 mm, the D3 of about 190 mm, the H1 of about 533 mm, the H2 of about 465 mm, and contains about 1200 L cell culture solution; and the oscillator has the rotational speed of about 25 rpm and the eccentricity of about 65 mm; wherein "about" refers to a range of the value ±20%.

10. A method for culturing cells without cell walls using the bioreactor system of claim 1, comprising:

calculating a rotational speed and an eccentricity of the oscillator required on the condition that a ratio of the total liquid surface area to the volume (S/V) of the culture solution is 5.65 or more, a turbulence kinetic energy is 2.73E-03 $m^2/s^2$ or more, and a flow field shear rate is 20.27/s or less when the culture solution achieves a steady state of eccentric motion through CFD simulation according to the shape of the bioreactor system and the volume of the culture solution, wherein the total liquid surface area is the sum of the contact area between the culture solution and the reactor wall surface and the contact area between the top surface and the gas; and adding the culture solution in the bioreactor system and inoculating cells, and configuring the oscillator according to the calculated rotational speed and eccentricity of the oscillator, and performing cell culture.

**FIG. 1**

**Point 1 is located at the lower dish and point 2 is located at the upper dish.**

**FIG. 2**

**FIG. 3**

**FIG. 4**

**FIG. 5**

**FIG. 6**

## INTERNATIONAL SEARCH REPORT

International application No.

**PCT/CN2020/113460**

### A. CLASSIFICATION OF SUBJECT MATTER

C12M 3/02(2006.01)i

According to International Patent Classification (IPC) or to both national classification and IPC

### B. FIELDS SEARCHED

Minimum documentation searched (classification system followed by classification symbols)

C12M

Documentation searched other than minimum documentation to the extent that such documents are included in the fields searched

Electronic data base consulted during the international search (name of data base and, where practicable, search terms used)

CNABS, CNTXT, CNKI, 万方, 百度学术: 细胞, 生物, 反应器, 锥, CFD, 计算流体力学, 计算流体动力学, 振荡, 摇动, 激流, 偏心, 偏心距, 转速, 频率, rpm, 湍动能, 剪切率, 液体总表面积; VEN, USTXT, EPTXT, WOTXT, Web of Science: cell, biology, reactor, bioreactor, conical, cone, CFD, computational fluid dynamics, shake, oscillate, eccentric, distance, eccentricity, rotate, speed, rate, frequency, rpm, turbulent kinetic energy, shear rate, liquid, solution, total, surface area

### C. DOCUMENTS CONSIDERED TO BE RELEVANT

| Category* | Citation of document, with indication, where appropriate, of the relevant passages | Relevant to claim No. |
|---|---|---|
| Y | 王康 (WANG, Kang). "振荡式生物反应器流场特性实验与模拟研究 (Experimental and Simulation Studies on Flow Field Characteristics of Shaking Bioreactors)" *中国优秀硕士学位论文全文数据库 基础科学辑 (Basic Sciences, China Master's Theses Full-Text Database)*, No. 7, 15 July 2019 (2019-07-15), ISSN: 1674-0246, pages 13-14, section 2.1, page 14, figure 2-2, pages 17-19, section 2.2.4, pages 19-20, section 2.3.1, pages 29-44, chapter 3 | 1-10 |
| Y | 李晶 等 (LI, Jing et al.). "摇瓶反应器中流体流动的CFD数值模拟 (Numerical Simulation of Fluid Flow in Erlenmeyer Shake Flask with Computational Fluid Dynamics)" *化工学报 (CIESC Journal)*, Vol. 60, No. 4, 30 April 2009 (2009-04-30), ISSN: 0438-1157, pages 882-883, section 4.5 | 1-10 |

☑ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 November 2020** | **30 November 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/113460** |

**C.     DOCUMENTS CONSIDERED TO BE RELEVANT**

| Category* | Citation of document, with indication, where appropriate, of the relevant passages | Relevant to claim No. |
| --- | --- | --- |
| Y | 常保安 (CHANG, Bao'an). "激流式生物反应器的流场仿真与结构优化 (The Flow Field Simulation and Structure Optimizaition of Shaking Bioreactor)" 中国优秀硕士学位论文全文数据库 基础科学辑 (Basic Sciences, China Master's Theses Full-Text Database), No. 5, 15 May 2014 (2014-05-15), ISSN: 1674-0246, pp. 9-38 | 1-10 |
| Y | LU,Zhiming et al. "CFD studies on hydrodynamic characteristics of shaking bioreactors with wide conical bottom" Journal of Chemical Technology and Biotechnology, Vol. 93, No. 3, 11 September 2017 (2017-09-11), ISSN: 0268-2575, pp. 811-816 | 1-10 |
| Y | 李晶 (LI, Jing). "典型生物反应器传质, 混合的计算流体力学模拟研究 (Computational Fluid Dynamics Simulation on Mass Transfer and Mixing in Typical Bioreactors)" 中国优秀硕士学位论文全文数据库 工程科技I辑 (China Master's Theses Full-Text Database, Engineering Science & Technology I), No. 5, 15 May 2010 (2010-05-15), ISSN: 1674-0246, pp. 17-20 | 1-10 |
| A | CN 108018204 A (ZHEJIANG JYSS BIO-ENGINEERING CO., LTD.) 11 May 2018 (2018-05-11) entire document | 1-10 |
| A | CN 206308363 U (ZHEJIANG JYSS BIO-ENGINEERING CO., LTD.) 07 July 2017 (2017-07-07) entire document | 1-10 |
| A | CN 101775430 A (EAST CHINA UNIVERSITY OF SCIENCE AND TECHNOLOGY) 14 July 2010 (2010-07-14) entire document | 1-10 |
| A | CN 101671712 A (EAST CHINA UNIVERSITY OF SCIENCE AND TECHNOLOGY) 17 March 2010 (2010-03-17) entire document | 1-10 |
| A | US 2012034695 A1 (SETHU, Palaniappan et al.) 09 February 2012 (2012-02-09) entire document | 1-10 |
| A | SARKAR,Jayati et al. "CFD of Mixing of Multi-Phase Flow in a Bioreactor Using Population Balance Model" Biotechnology Progress, Vol. 32, No. 3, 29 February 2016 (2016-02-29), ISSN: 8756-7938, pp. 613-628 | 1-10 |
| A | 张建文 等 (ZHANG, Jianwen et al.). "机械搅拌生物反应器的CFD模拟及剪切力对红花细胞悬浮培养的影响 (CFD Simulation of a Mechanically Stirred Bioreactor and Effect of Shear Stress on Suspension Cultures of Carthamus tinctorius)" 华东理工大学学报 (自然科学版) (Journal of East China University of Science and Technology (Natural Science Edition)), Vol. 42, No. 4, 31 August 2016 (2016-08-31), ISSN: 1006-3080, pp. 492-498 | 1-10 |

## INTERNATIONAL SEARCH REPORT
### Information on patent family members

International application No.

**PCT/CN2020/113460**

| Patent document cited in search report | | | Publication date (day/month/year) | Patent family member(s) | | | Publication date (day/month/year) |
|---|---|---|---|---|---|---|---|
| CN | 108018204 | A | 11 May 2018 | WO | 2018082552 | A1 | 11 May 2018 |
| | | | | EP | 3532601 | A1 | 04 September 2019 |
| CN | 206308363 | U | 07 July 2017 | None | | | |
| CN | 101775430 | A | 14 July 2010 | CN | 104774902 | A | 15 July 2015 |
| CN | 101671712 | A | 17 March 2010 | CN | 101671712 | B | 18 September 2013 |
| US | 2012034695 | A1 | 09 February 2012 | None | | | |

Form PCT/ISA/210 (patent family annex) (January 2015)